(19) Europäisches Patentamt
European Patent Office
Office européen des brevets

(11) Veröffentlichungsnummer: **0 053 276**
**A2**

(12) # EUROPÄISCHE PATENTANMELDUNG

(21) Anmeldenummer: **81108965.5**

(22) Anmeldetag: **27.10.81**

(51) Int. Cl.³: **C 07 D 501/20**, A 61 K 31/545

(30) Priorität: **02.12.80 DE 3045331**
**02.12.80 DE 3045330**

(43) Veröffentlichungstag der Anmeldung: **09.06.82**
**Patentblatt 82/23**

(84) Benannte Vertragsstaaten: **AT BE CH DE FR IT LI LU NL SE**

(71) Anmelder: **Dr. Karl Thomae GmbH, Postfach 1755, D-7950 Biberach (Riss) (DE)**

(72) Erfinder: **Wetzel, Bernd, Dr. Dipl.-Chem., Kapellenweg 21, D-7950 Biberach 1 (DE)**
Erfinder: **Woitun, Eberhard, Dr, Dipl.-Chem., Stecherweg 17, D-7950 Biberach 1 (DE)**
Erfinder: **Reuter, Wolfgang, Dr. Dipl.-Chem., Nelkenweg 10, D-7951 Laupertshausen (DE)**
Erfinder: **Maier, Roland, Dr. Dipl.-Chem., Bodelschwinghstrasse 39, D-7950 Biberach 1 (DE)**
Erfinder: **Lechner, Uwe, Dr., Panoramastrasse 12, D-7951 Ummendorf (DE)**
Erfinder: **Goeth, Hanns, Dr., Oberer Bühl 6, D-7950 Biberach 1 (DE)**

(54) **Neue Cephalosporine, Verfahren zu ihrer Herstellung und diese Verbindungen enthaltende Arzneimittel.**

(57) Beschrieben werden neue Cephalosporine der allgemeinen Formel I

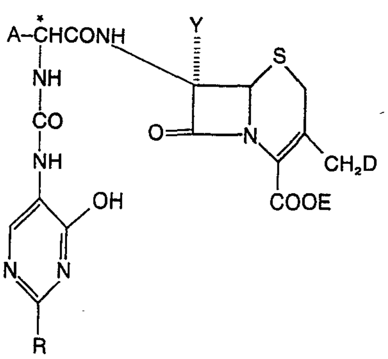

(I)

worin Y Wasserstoff oder Methoxy bedeutet,

A sofern Y die Methoxygruppe darstellt, die Phenyl-, 4-Hydroxyphenyl-, 3,4-Dihydroxyphenyl- oder die 2- oder 3-Thienylgruppe bedeutet oder, sofern Y Wasserstoff oder die Methoxygruppe darstellt, eine Gruppe der Formel

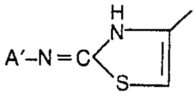

worin A′ Wasserstoff, eine Halogenacetyl-, die Formyl- oder Tritylgruppe bedeutet,

D Wasserstoff, die Acetoxy- oder Aminocarbonyloxygruppe, die Pyridinium- oder 4-Aminocarbonylpyridiniumgruppe oder die Gruppe –SHet bedeutet, worin Het ein gegebenenfalls substituierter Thiadiazol-, 4H-5,6-Dioxo-1,2,4-triazinyl- oder ein Tetrazolylrest darstellt,

R die Gruppe $-NHR_2$ bedeutet, worin $R_2$ ein gegebenenfalls substituierter Pyridyl-, Pyrimidinyl-, Thienyl-, Furyl-, Thienylmethyl-, Imidazolylmethyl-, Thiazolylmethyl-, Pyridylmethyl- oder Pyrimidinylmethylrest sein kann und

E Wasserstoff oder eine Schutzgruppe bedeutet, und ihre Salze mit anorganischen oder organischen Basen.

Die Verbindungen besitzen eine sehr starke antibakterielle Wirkung.

EP 0 053 276 A2

ACTORUM AG

COMPLETE DOCUMENT

0053276

Case 5/812
Dr. Bu/fra

DR. KARL THOMAE GMBH, BIBERACH AN DER RISS

Neue Cephalosporine, Verfahren zu ihrer Herstellung und diese Verbindungen enthaltende Arzneimittel

Die Erfindung betrifft neue Cephalosporine der allgemeinen Formel I

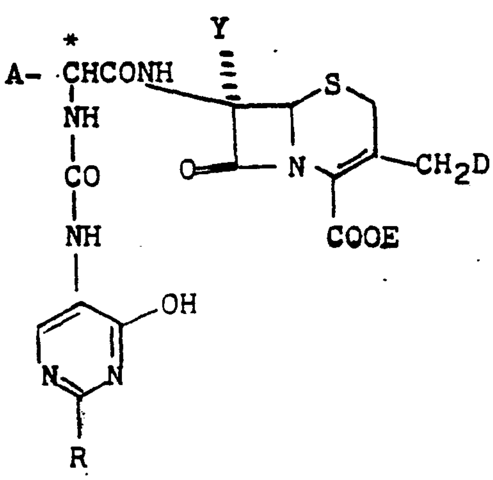

,(I)

bzw. deren mögliche Tautomere, ihre physiologisch verträglichen Salze mit anorganischen oder organischen Basen, Verfahren zur Herstellung dieser Verbindungen und diese Verbindungen enthaltende Arzneimittel.

In der allgemeinen Formel I bedeutet
Y ein Wasserstoffatom oder die Methoxygruppe und
A sofern Y die Methoxygruppe darstellt, die Phenyl-, 4-Hydroxyphenyl-, 3,4-Dihydroxyphenyl-, die 2- oder 3-Thienylgruppe, oder, sofern Y ein Wasserstoffatom oder die Methoxygruppe bedeutet, eine Gruppe der Formel

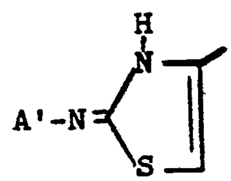

wobei in dieser Gruppe

A' ein Wasserstoffatom, die Gruppe $-COCH_2Cl$, $-COCH_2Br$, $-COOCH_2CCl_3$; die Formyl- oder die Tritylgruppe darstellt,

D ein Wasserstoffatom, die Acetoxy- oder Aminocarbonyloxygruppe, die Pyridinium- oder 4-Aminocarbonylpyridiniumgruppe oder die Gruppe SHet, wobei Het die 1,3,4-Thiadiazol-5-yl-, 2-Methyl-1,3,4-thiadiazol-5-yl-, die 1,2,4-Thiadiazol-5-ylgruppe, die 3-Methyl-1,2,4-thiadiazol-5-ylgruppe, die 4H-5,6-Dioxo-1,2,4-triazin-3-yl- oder 4-Methyl-5,6-dioxo-1,2,4-triazin-3-ylgruppe, die 1-Vinyl-tetrazol-5-yl- oder 1-Allyltetrazol-5-ylgruppe oder eine Gruppe der allgemeinen Formel II,

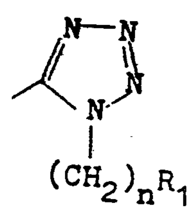

,(II)

in der n die Zahlen 1 bis 3 bedeutet und $R_1$ für die Hydroxygruppe, die Amino-, Dimethylamino-, Acetylamino-, Aminocarbonyl-, Aminocarbonylamino-, Aminosulfonyl-, Aminosulfonylamino-, Methylcarbonyl-, Methylsulfonylamino-, Cyano-, Hydroxysulfonylamino-, Methylsulfonyl-, Methylsulfinyl-, sowie für eine Carbonsäure- oder Sulfonsäuregruppe steht, oder in der $(CH_2)_nR_1$ eine Alkylgruppe mit 1 bis 4 Kohlenstoffatomen oder einen 2,3-Dihydroxypropylrest bedeuten,

R eine Gruppe der allgemeinen Formel $NHR_2$, in der $R_2$ eine substituierte oder unsubstituierte 3-Pyridyl-, 5-Pyrimidinyl-, 2-Thienyl-, 2-Furylmethyl-, 2-Thienylmethyl-, 2-Imidazolylmethyl-, 2-Thiazolylmethyl-, 3-Pyridylmethyl- oder eine 5-Pyrimidinylmethylgruppe bedeutet, wobei diese Gruppen durch ein Chloratom, eine Methyl-, Acetylamino-, Hydroxy-, Methylsulfinyl-, Methylsulfonyl-, Aminocarbonyl- oder Aminosulfonylgruppe substituiert sein können,

E bedeutet ein Wasserstoffatom oder eine in vitro oder in vivo leicht spaltbare Carboxylschutzgruppe. Als Carboxylschutzgruppen kommen im Sinne der Erfindung solche in Frage, welche auch bisher schon auf dem Gebiet der Penicilline und Cephalosporine eingesetzt wurden, insbesondere esterbildende Gruppen, die durch Hydrogenolyse oder Hydrolyse oder andere Behandlungen unter milden Bedingungen entfernt werden können oder esterbildende Gruppen, welche leicht im lebenden Organismus abgespalten werden können.

Beispiele für in vitro leicht spaltbare Schutzgruppen sind z.B. die Benzyl-, Diphenylmethyl-, Trityl-, t-Butyl-, die 2,2,2-Trichloräthyl- oder die Trimethylsilylgruppe. Beispiele für in vivo leicht spaltbare Schutzgruppen sind z.B. Alkanoyloxyalkylgruppen, wie z.B. die Acetoxymethyl-, Propionyloxymethyl-, 2-Acetoxy-äthyl- oder Pivaloyloxymethylgruppe oder die Phthalidylgruppe. Bedeutet E ein

Wasserstoffatom, so fallen unter den Anspruch auch pharmakologisch verträgliche Salze mit anorganischen oder organischen Basen, wie z.B. die Alkali- oder Erdalkalisalze, z.B. die Natrium-, Kalium-, Magnesium- oder Calciumsalze, die Ammoniumsalze, oder organische Aminsalze, z.B. solche mit Triäthylamin oder Dicyclohexylamin.

Das Sternchen an dem Kohlenstoffatom in den Verbindungen der allgemeinen Formel I bedeutet ein Asymmetriezentrum.

Steht D für Pyridinium oder Aminocarbonylpyridinium, so trifft für diese Verbindungen die allgemeine Formel Ia zu:

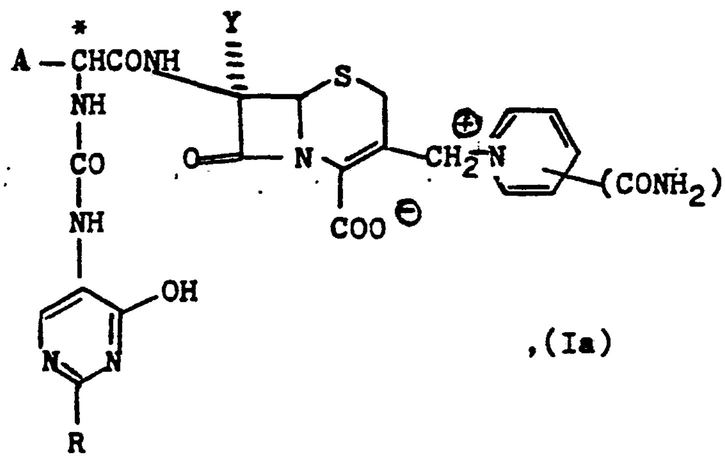

,(Ia)

Besonders bevorzugt sind diejenigen Verbindungen der allgemeinen Formel I, worin

A die oben angegebene Bedeutung hat, wobei A' für Wasserstoff steht

Y und R die oben angegebenen Bedeutungen haben und

D für die Acetoxygruppe steht, oder eine Gruppe SHet darstellt, in der Het die 2-Methyl-1,3,4-thiadiazol-5-yl-gruppe, die 4-Methyl-5,6-dioxo-1,2,4-triazin-3-yl-gruppe oder eine Tetrazol-5-yl-gruppe der Formel II bedeutet, in der der Substituent $R_1$ und n die oben angegebene Bedeutung haben.

Die Cephalosporinverbindungen der allgemeinen Formel I und die nachstehend beschriebenen Zwischenprodukte können in 2 tautomeren Formen bezüglich des Pyrimidinrings vorliegen. Es hängt besonders vom jeweiligen Lösungsmittel und von der Art des Substituenten R ab, welche der nachstehenden Formen I oder I' überwiegt:

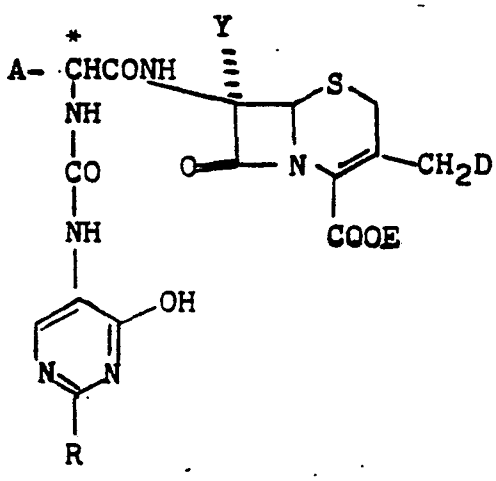

,(I)

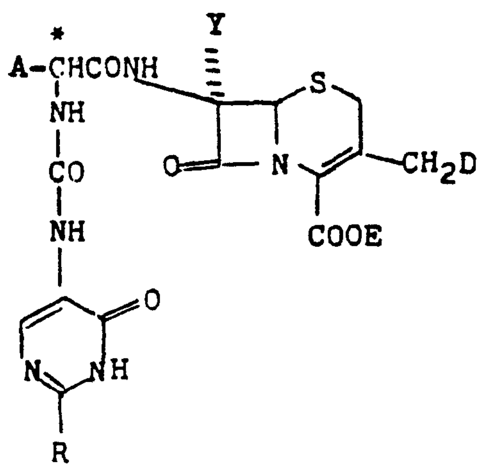

I'

Ebenso können, wenn A für Aminothiazol steht, bezüglich dieses Ringes die folgenden tautomeren Formen auftreten:

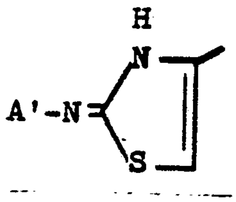

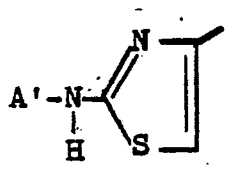

Es versteht sich von selbst, daß die eingangs angegebenen Verbindungen der allgemeinen Formel I immer alle Tautomeren umfassen.

Die Verbindungen der allgemeinen Formel I können bezüglich des mit einem Sternchen gekennzeichneten Chiralitätszentrums C+ in den beiden möglichen R und S-Konfigurationen, jedoch auch als ein Gemisch dieser Konfigurationen vorliegen.

Die Verbindungen der allgemeinen Formel I können wie folgt hergestellt werden:

1. Durch Umsetzung eines 7-Aminocephalosporansäurederivates der allgemeinen Formel III,

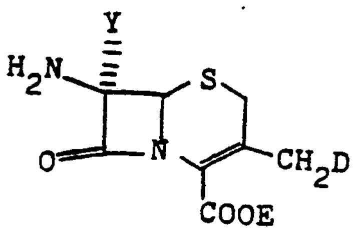

,(III)

in der D ein Wasserstoffatom, die Acetoxy- oder Aminocarbonyloxygruppe oder die Gruppe -SHet bedeutet und E und Y die oben genannten Bedeutungen haben, mit Ureidocarbonsäuren der allgemeinen Formel IV,

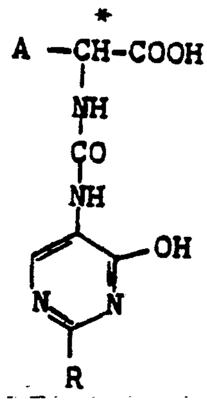

,(IV)

in der A und R die vorstehende Bedeutung haben, oder ihren Salzen oder reaktiven Derivaten.

Als reaktive Derivate der Ureidocarbonsäuren der allgemeinen Formel IV kommen bespielsweise deren Säureanhydride wie z.B. die, die sich von Chlorameisensäureestern, z.B. Chlorameisensäureäthyl- oder -isobutylester, ableiten, oder deren reaktive Ester, wie der p-Nitrophenylester oder der N-Hydroxysuccinimidester, oder deren reaktive Amide, wie das N-Carbonylimidazol, aber auch deren Säurehalogenide, wie das entsprechende Säurechlorid oder deren Säureazide in Frage. Prinzipiell können jedoch alle Verknüpfungsmethoden, wie sie aus der ß-Lactamchemie bekannt sind, verwendet werden.

Die Ureidocarbonsäure, ihre Salze oder ihre reaktiven Derivate werden mit den 7-Aminocephalosporansäurederivaten in einem Lösungsmittel bei Temperaturen zwischen -40°C und +40°C, gegebenenfalls in Gegenwart einer Base, umgesetzt. Wird z.B. ein Anhydrid der Ureidocarbonsäure, beispielsweise das Anhydrid mit dem Äthylchloroformiat eingesetzt, so wird die Reaktion unter Kühlung, beispielsweise bei -10° bis +10°C in einem Lösungsmittel, wie Aceton, Tetrahydrofuran, Dimethylformamid, Chloroform, Dichlormethan,

Hexametapol oder in einem Gemisch dieser Lösungsmittel, durchgeführt. Setzt man beispielsweise einen N-Hydroxysuccinimidester der Ureidocarbonsäure mit Derivaten der allgemeinen Formel III um, so wird die Reaktion vorzugsweise bei 0 bis 20°C in Anwesenheit einer Base, wie z. B. Triäthylamin, in einem Lösungsmittel wie Dimethylformamid, Dichlormethan, Dioxan oder in einem Gemisch solcher Lösungsmittel durchgeführt.

Die Umsetzung einer Ureidocarbonsäure der allgemeinen Formel IV selbst oder ihrer Salze mit Verbindungen der allgemeinen Formel III erfolgt vorteilhafterweise in Gegenwart eines Kondensationsmittels, z. B. in Gegenwart von N,N'-Dicyclohexylcarbodiimid.

2. Durch Umsetzung von 7-Aminocephalosporansäuren der allgemeinen Formel V oder ihrer Salze mit anorganischen oder organischen Säuren,

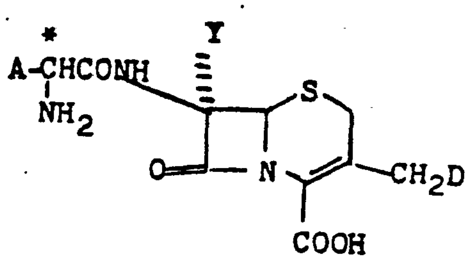 ,(V)

bei denen A, D und Y die oben angegebenen Bedeutungen haben, mit einem Pyrimidinderivat der allgemeinen Formel VI,

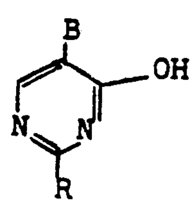 ,(VI)

in der R wie oben definiert ist und B die Gruppe -NCO oder ein reaktives Derivat der Gruppe -NHCOOH bedeutet, wie z.B.

die Gruppen -NHCOCl, -NHCOBr oder -NH-COO-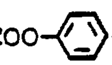-$NO_2$,

wobei die Gruppe-NHCOCl besonders bevorzugt ist. Es können auch Gemische von solchen Pyrimidinderivaten der allgemeinen Formel VI verwendet werden, in der B teils die eine und teils die andere der vorstehend genannten Bedeutungen besitzt, z.B. die Gruppen -NCO und $-\underset{H}{N}-COCl$ gleichzeitig nebeneinander.

Die Reaktion wird bevorzugt in beliebigen Mischungen von Wasser mit solchen organischen Lösungsmitteln, die mit Wasser mischbar sind, wie Ketonen, z.B. Aceton, cyclische Äther, z.B. Tetrahydrofuran oder Dioxan, Nitrilen, z.B. Acetonitril, Formamiden, z.B. Dimethylformamid, Dimethylsulfoxid oder Alkoholen z.B. Isopropanol oder in Hexametapol durchgeführt. Besonders bevorzugt ist ein Gemisch aus Tetrahydrofuran und Wasser. Dabei hält man den pH der Reaktionsmischung durch Zusatz von Basen oder Verwendung von Pufferlösungen in einem pH-Bereich von etwa 2,0 bis 9,0, vorzugsweise zwischen pH 6,5 und 8,0.

3. Durch Umsetzung einer Verbindung der allgemeinen Formel VII,

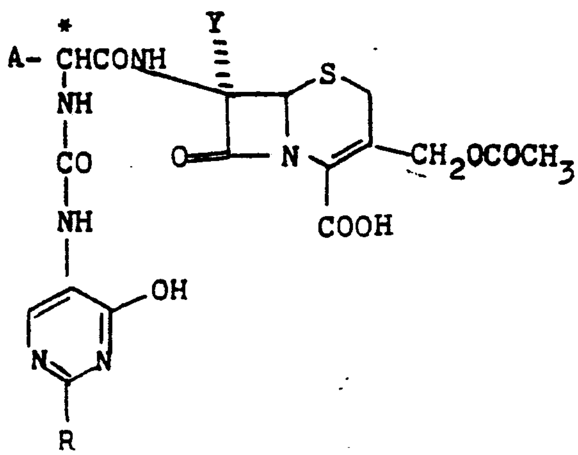
,(VII)

in der A, Y und R die oben angegebenen Bedeutungen haben, mit Pyridin oder 4-Aminocarbonylpyridin oder mit einer Verbindung der allgemeiner Formel VIII,

Het-S-M ,(VIII)

in der Het die oben angegebenen Bedeutungen hat und M für ein Wasserstoffatom oder ein Alkalimetall oder ein Erdalkalimetall steht. Dazu wird z.B. eine Verbindung der Formel VII mit beispielsweise 1-Methyl-5-mercapto-1,2,3,4-tetrazol in einem Lösungsmittel, z.B. Wasser, Methanol, Äthanol, Aceton, Methyläthylketon, Tetrahydrofuran, Acetonitril, Essigsäureäthylester, Dimethoxyäthan, Dimethylformamid, Dimethylsulfoxid, Chloroform oder einem Gemisch dieser Lösungsmittel umgesetzt. Vorzugsweise wird ein stark polares Lösungsmittel, wie Wasser verwendet: In diesem Fall wird der pH-Wert der Reaktionslösung vorteilhafterweise auf 2-10 und insbesondere auf 4-8 gehalten. Der gewünschte pH-Wert kann durch Zugabe einer Pufferlösung, wie Natriumphosphat, eingestellt werden. Die Reaktionsbedingungen unterliegen keinen besonderen Beschränkungen. Normalerweise wird die Umsetzung bei einer Temperatur im Bereich von $0^{o}$ bis $100^{o}C$ während einer Zeitdauer von einigen Stunden durchgeführt. Man erhält Verbindungen der allgemeinen Struktur I, in der D für SHet oder für den Pyridinium- oder 4-Aminocarbonylpyridiniumrest steht.

4. Eine Verbindung der allgemeinen Formel I, in der Y die Methoxygruppe bedeutet, kann weiter dadurch erhalten werden, daß man eine Verbindung der allgemeinen Formel IX, in der

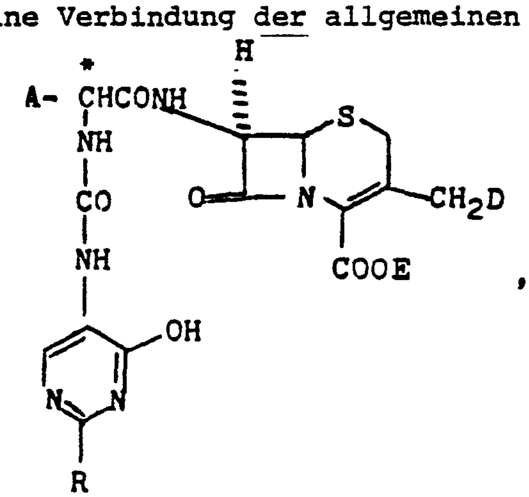

,(IX)

A, R, D und E die oben angegebene Bedeutung haben in Anwesenheit von Methanol mit einem Alkalimetall-Methylat der allgemeinen Formel $M^{+}OCH_3^{-}$, worin $M^{+}$ ein Alkalimetallion

bedeutet, und dann mit einem Halogenierungsmittel umsetzt. Dazu wird ein Cephalosporin der allgemeinen Formel IX in einem inerten Lösungsmittel, z.B. Tetrahydrofuran, Dioxan, Äthylenglycoldimethyläther, Methylenchlorid, Chloroform, Dimethylformamid, Methanol oder dgl. oder in einem Gemisch von zweien dieser Lösungsmittel aufgelöst oder suspendiert. Zu der erhaltenen Lösung oder Suspension gibt man ein Alkalimetallmethylat zusammen mit Methanol. Das erhaltene Gemisch wird zur Reaktion gebracht und das Reaktionsgemisch wird dann mit einem Halogenierungsmittel umgesetzt. Bei dieser Reaktion verwendet man Methanol im Überschuß und die Menge des Alkalimetallmethylats beträgt vorzugsweise 1 bis 8 Äquivalente pro Äquivalent des verwendeten Cephalosporins. Der Ausdruck "im Überschuß" bedeutet eine Menge von mehr als einem Äquivalent pro Äquivalent des Cephalosporins. Alle Reaktionen werden bei -120 bis -10°C und vorzugsweise -100°C bis -50°C durchgeführt. Eine Reaktionszeit von 5 bis 60 Min. reicht aus. Die Reaktion wird durch Ansäuern des Reaktionssystems abgebrochen.

Das bei diesem Verfahren eingesetzte Halogenierungsmittel ist allgemein als Quelle für positive Halogenatome, z.B. $Cl^+$ oder $Br^+$, $J^+$ bekannt. Beispiele solcher Halogenierungsmittel sind Halogen, wie Chlor, Brom usw.; N-Halogenimide, wie N-Chlor-succinimid, N-Bromsuccinimid und dgl.; N-Halogenamide wie N-Chloracetamid, N-Bromacetamid, usw.; N-Halogensulfonamide, wie N-Chlorbenzolsulfonamid, N-Chlor-p-toluolsulfonamid usw.; 1-Halogenbenzotriazole; 1-Halogentriazine, organische Hypohalogenite, wie tert.-Butylhypochlorit, tert.-Butylhypojodit usw.; Halogenhydantoine, wie N,N-Dibromhydantoin, usw.. Unter diesen Halogenierungsmitteln ist tert.-Butylhypochlorit bevorzugt. Das Halogenierungsmittel wird in einer Menge eingesetzt, welche ausreicht, eine zur Menge des Cephalosporins der allgemeinen Formel IX äquivalente Menge positives Halogen abzugeben.

Geeignete Säuren für das Abbrechen der Reaktion sind solche, welche bei Zusatz zum kalten Reaktionsgemisch nicht zu einer Verfestigung des Reaktionsgemisches oder zum Gefrieren des Reaktionsgemisches zu einer schweren viskosen Masse führen. Beispiele geeigneter Säuren sind 98%-ige Ameisensäure, Eisessig, Trichloressigsäure und Methansulfonsäure. Nach dem Abbrechen der Reaktion wird das überschüssige Halogenierungsmittel durch Behandlung mit einem Reduktionsmittel, z.B. Trialkylphosphit, Natriumthiosulfat oder dgl. entfernt.

Die nach den vorstehenden Verfahren hergestellten Verbindungen der allgemeinen Formel I, in der E einen anderen Rest als ein Wasserstoffatom bedeutet, können in an sich bekannter Weise zur Abspaltung der Schutzgruppe behandelt werden. Es werden dadurch die Verbindungen erhalten, in denen E Wasserstoff bedeutet und die im Sinne der Erfindung als ganz besonders bevorzugte Endverbindungen gelten. Beispielsweise wird eine Verbindung der allgemeinen Formel I, in der E für eine Diphenylmethylgruppe steht, in bekannter Weise mit Anisol und Trifluoressigsäure zur Abspaltung der Esterschutzgruppe behandelt oder es kann in ebenfalls bekannter Weise eine Silylschutzgruppe durch wäßrige Hydrolyse entfernt werden.

Die nach den vorstehenden Verfahren hergestellten Verbindungen der allgemeinen Formel I, in der A für Aminothiazolyl- steht und A' einen anderen Rest als ein Wasserstoffatom bedeutet, können in an sich bekannter Weise zur Abspaltung der Iminoschutzgruppe behandelt werden. Es werden dadurch die Verbindungen erhalten, in denen A' Wasserstoff bedeutet und die im Sinne der Erfindung als ganz besonders bevorzugte Endverbindungen gelten. Beispielsweise wird eine Verbindung der allgemeinen Formel I, in der A für die Aminothiazolylgruppe, A' für die Chloracetylgruppe und E für eine Diphenylmethylgruppe steht, zunächst mit Thioharnstoff zur Abspaltung der Chloracetylgruppe und sodann in bekannter Weise mit Anisol und Trifluoressigsäure zur Abspaltung der Esterschutzgruppe behandelt (vgl. auch DT-OS 2 924 296), oder es kann die

Esterschutzgruppe zunächst abgespalten werden und anschließend die Chloracetylschutzgruppe mit Natrium-N-Methyldithiocarbamat entfernt werden (vgl. EP-OS Nr. 2586).

Die Verbindungen der allgemeinen Formel I, in der E ein Natrium- oder Kaliumkation darstellt, werden durch Umsetzung der entsprechenden freien Säuren der Verbindungen der allgemeinen Formel I, in der E ein Wasserstoffatom darstellt, mit dem entsprechenden salzbildenden Ion hergestellt. Hierzu eignet sich zum Beispiel die in der Chemie der Penicilline und Cephalosporine übliche Umsetzung mit Natriumäthylhexanoat, oder die Umsetzung mit Natriumhydrogencarbonat und anschließende Gefriertrocknung. Die Cephalosporinantibiotika der allgemeinen Formel I, in der E ein Wasserstoffatom bedeutet, können auf bekannte Weise in die Acyloxyalkylester, worin E z.B. einen Pivaloyloxymethylrest

$$-CH_2-O\underset{\underset{O}{\|}}{C}-C(CH_3)_3$$

darstellt, überführt werden, indem man ein Alkalisalz der Cephalosporincarbonsäure, beispielsweise ein Natrium- oder Kaliumsalz, mit einem Pivaloyloxymethylhalogenid der Formel

$$Hal-CH_2-O-\underset{\underset{O}{\|}}{C}-C(CH_3)_3,$$

worin Hal für Chlor, Brom oder Jod steht, umsetzt. Weitere geeignete Acyloxyalkylhalogenide sind z.B. Chlormethylacetat, Brommethylpropionat oder 1-Bromäthylacetat.

Bei Verwendung der entsprechenden Ausgangsverbindungen ist es möglich, die Verbindungen der allgemeinen Formel I in Form der Racemate oder in Form der einzelnen Isomeren herzustellen. Wenn das Endprodukt in der D,L-Form anfällt, gelingt es, die reinen D- und L-Diastereoisomeren durch präparative Flüssigkeitschromatographie (HPLC) herzustellen. Die Erfindung betrifft die Racemate und die Isomeren.

Die Ureidocarbonsäuren der allgemeinen Formel IV sind, wenn A nicht Aminothiazolyl bedeutet, literaturbekannt. Sie werden in der DT-OS 29 24 296 beschrieben. Für A = Aminothiazolyl werden sie erhalten, wenn man das Aminosäurederivat der allgemeinen Formel X oder seine Salze mit Säuren, z. B. mit $CF_3COOH$

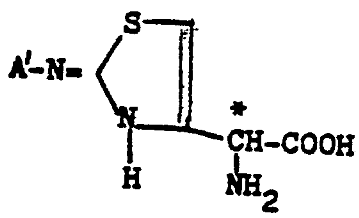

,(X)

mit einem Pyrimidinderivat der allgemeinen Formel VI

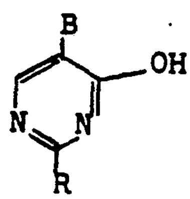

,(VI)

umsetzt.

Die Umsetzung erfolgt bei Temperaturen zwischen $-20^{\circ}$ und $+40^{\circ}C$, vorzugsweise zwischen $0^{\circ}$ und $+20^{\circ}C$, in einem Lösungsmittel. Als Lösungsmittel können hierbei z.B. Gemische von Wasser und organischen Lösungsmitteln, die mit Wasser mischbar sind, verwendet werden, beispielsweise Aceton, Tetrahydrofuran, Dioxan, Acetonitril, Dimethylformamid, Äthanol, Dimethylsulfoxid. Gegebenenfalls wird die Verwendung eines halogenwasserstoffbindenden Mittels notwendig, als solche eignen sich beispielsweise Trialkylamine, wie Triäthylamin oder anorganische Basen, wie verdünnte Natronlauge.

Die Ausgangsstoffe der allgemeinen Formel VI können beispielsweise durch Umsetzung der entsprechenden 5-Aminopyrimidine der allgemeinen Formel XI

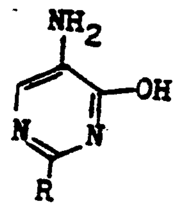 ,(XI)

in der R wie oben definiert ist, mit Phosgen gewonnen werden. Diese Umsetzung erfolgt vorzugsweise in einem nicht Hydroxylgruppen-enthaltenden Lösungsmittel, wie Tetrahydrofuran, Methylenchlorid, Chloroform, Dimethoxyäthan oder Hexametapol bei Temperaturen zwischen -40° und +60°C, vorzugsweise zwischen -10° und +20°C. Dabei empfiehlt es sich, den entstehenden Chlorwasserstoff durch äquimolare Mengen einer inerten organischen Base, wie Triäthylamin oder Pyridin, zu binden. Als Lösungsmittel kann auch Pyridin im Überschuß verwendet werden. Sollten die betreffenden Aminopyrimidine der allgemeinen Formel XI in einem der erwähnten Lösungsmittel schwer löslich sein, kann die Phosgenierung auch in heterogener Phase durchgeführt werden. Des weiteren können in einer besonders bevorzugten Ausführungsform die Aminopyrimidine der allgemeinen Formel XI durch Behandlung mit einem Silylierungsmittel, wie Hexamethyldisilazan, Trimethylchlorsilan/Triäthylamin, Trimethylsilyldiäthylamin oder N,O-Bis-trimethylsilylacetamid in ein im allgemeinen in den erwähnten Lösungsmitteln sehr leicht lösliches, einfach oder, entsprechend den vorhandenen austauschbaren Wasserstoffatomen, mehrfach silyliertes Aminopyrimidin überführt werden, das mit Phosgen dann zu den entsprechenden Verbindungen der allgemeinen Formel VI reagiert, wobei bevorzugt ohne Basenzusatz gearbeitet wird. Je nach der Art des Lösungsmittels, der Höhe der Temperatur, der Menge und Art einer eventuell eingesetzten Base entsteht entweder überwiegend das entsprechende Isocyanat oder Carbaminsäurehalogenid oder ein Gemisch dieser beiden Verbindungen. Je nach den Bedingungen liegt das Isocyanat der allgemeinen Formel VI auch teilweise oder ganz als ein den Isocyanaten isomeres 1-(H)-Oxazolo/5,4-d/pyrimidin-2-on der allgemeinen Formel VIa

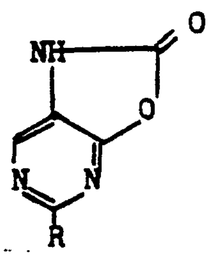

,(VIa)

oder als ein je nach Art des Substituenten R ein oder mehrfach silyliertes Analoges vor.

Die durch Phosgenierung entstandenen Ausgangsprodukte der allgemeinen Formel VI bzw. deren Gemische oder deren Silylprodukte sind in den obenerwähnten Lösungsmitteln im allgemeinen gut löslich und können, nach Entfernung des überschüssigen Phosgens, ohne weitere Reinigung mit den entsprechenden Cephalosporinderivaten der allgemeinen Formel V direkt umgesetzt werden. Es ist jedoch auch möglich, das Zwischenprodukt der allgemeinen Formel VIa zu isolieren, es gegebenenfalls mit einem protischen Lösungsmittel zu entsilylieren, z.B. mit Methanol oder Wasser, es erforderlichenfalls auf Grund seiner Löslichkeitseigenschaften zu reinigen und in der obendargestellten Weise umzusetzen.

Synthesen für 2-substituierte 5-Amino-4-hydroxy-pyrimidine der allgemeinen Formel XI sind prinzipiell literaturbekannt. Spezielle Beispiele werden in der DT-OS 29 28 344 beschrieben. Die Synthese von Ausgangsprodukten der allgemeinen Formel V ist literaturbekannt. Dazu wird, z. B. wenn A die Aminothiazolylgruppe bedeutet, ein Cephalosporinderivat der allgemeinen Formel III mit einer an der Aminogruppe geschützten Aminosäure der allgemeinen Formel X unter den in der Cephalosporinchemie üblichen Bedingungen umgesetzt und anschließend die Schutzgruppen wie üblich abgespalten (vgl. DT-OS 29 24 296).

Die 7-Aminocephalosporansäurederivate der allgemeinen Formel III sind literaturbekannt.

Die Cephalosporine der allgemeinen Formel VII, die als Ausgangsprodukte verwendet werden können, lassen sich nach der im experimentellen Teil beschriebenen Methode herstellen.

Die Herstellung der als Ausgangsprodukte verwendeten Cephalosporine der allgemeinen Formel IX wird, sofern A für Aminothiazol steht, ebenfalls im experimentellen Teil beschrieben. Sofern A für eine andere erfindungsgemäße Gruppe als Aminothiazol steht, wird ihre Herstellung in der DE-OS 29 28 344 oder DE-OS P 30 47 082.7 dargestellt.

Es wurde gefunden, daß die Verbindungen der allgemeinen Formel I wertvolle pharmakologische Eigenschaften bei guter Verträglichkeit besitzen. Die erfindungsgemäßen Wirkstoffe können daher zur Prophylaxe und Chemotherapie von lokalen und systemischen Infektionen in der Human- und Tiermedizin verwendet werden. Als Krankheiten, die durch die erfindungsgemäßen Verbindungen verhindert bzw. geheilt werden können, seien beispielsweise solche der Atmungswege, des Rachenraumes und der Harnwege genannt; die Verbindungen wirken insbesondere gegen Pharyngitis, Pneumonie, Peritonitis, Pyelonephritis, Otitis, Cystitis, Endocarditis, Bronchitis, Arthritis und allgemeine systemische Infektionen. Weiter können diese Verdungen als Stoffe zur Konservierung von anorganischen oder organischen Materialien verwendet werden, besonders von organischen Materialien, wie Polymeren, Schmiermittel, Farben, Fasern, Leder, Papier und Holz sowie von Lebensmitteln.

Dieses wird dadurch ermöglicht, daß die Verbindungen der allgemeinen Formel I sowohl in vitro als auch in vivo gegen schädliche Mikroorganismen, insbesondere gegen grampositve und gramnegative Bakterien und bakterienähnliche Mikroorganismen sehr stark wirken, wobei sie sich besonders durch ein breites Wirkungsspektrum auszeichnen.

Mit diesen Cephalosporinderivaten können beispielsweise lokale und/oder systemische Erkrankungen behandelt und/oder verhindert werden, die durch die folgenden Erreger oder durch Mischungen der folgenden Erreger verursacht werden:

Micrococcaceae, wie Staphylokokken;
Lactobacteriaceae, wie Streptokokken;
Neisseriaceae, wie Neisserien;
Corynebacteriaceae, wie Corynebakterien;
Enterobacteriaceae, wie Escherichiae-Bakterien der Coli-Gruppe,
Klebsiella-Bakterien, z.B. K. pneumoniae;
Proteae-Bakterien der Proteus-Gruppe z.B. Proteus vulgaris;
Salmonella-Bakterien, z.B. S.thyphimurium;
Shigella-Bakterien, z.B. Shigella dysenteriae;
Pseudomonas-Bakterien, z.B. Pseudomonas aeruginosa;
Aeromonas-Bakterien, z.B. Aeromonas lique faciens;
Spirillaceae, wie Vibrio-Bakterien, z.B. Vibrio cholerae;
Parvobacteriaceae oder Brucellaceae, wie Pasteurella-Bakterien;
Brucella-Bakterien, z.B. Brucella abortus;
Haemophilus-Bakterien, z.B. Haemophilus influenzae;
Bordetella-Bakterien, z.B. Bordetella pertussis;
Moraxella-Bakterien, z.B. Moraxella lacunata;
Bacteroidaceae, wie Bacteroides-Bakterien;
Fusiforme-Bakterien, z.B. Fusobacterium fusiforme;
Sphaerophorus-Bakterien, z.B. Sphaerophorus necrophorus;
Bacillaceae, wie aerobe Sporenbildner, z.B. Bacillus anthracis;
anaerobe Sporenbildner-Chlostridien, z.B. Chlostridium perfringens;
Spirochaetaceae, wie Borrelia-Bakterien;
Treponema-Bakterien, z.B. Treponema pallidum;
Leptospira-Bakterien, wie Leptospira interrogans.

Die obige Aufzählung von Erregern ist lediglich beispielhaft und keinesweges beschränkend aufzufassen.

In der folgenden Tabelle 1 werden einige typische, besonders gut wirksame Verbindungen, entsprechend der vorliegenden Erfindung, aufgezählt:

Tabelle 1 Cephalosporine der allgemeinen Formel bzw. ihre Natrium-Salze

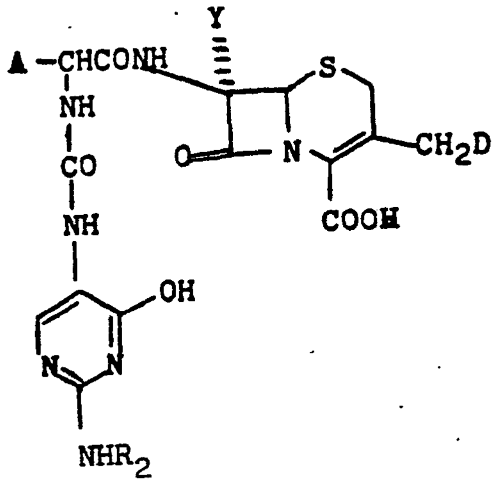

| | A | $R_2$ | Y | D |
|---|---|---|---|---|
| 1 | 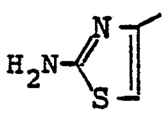 | 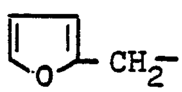 | H | 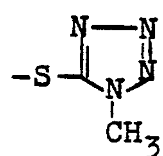 |
| 2 | 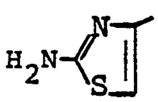 | 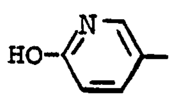 | H | 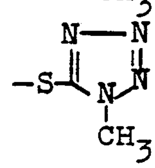 |
| 3 | 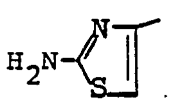 | 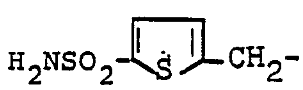 | H | $-OCOCH_3$ |
| 4 | 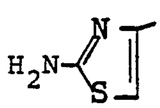 | 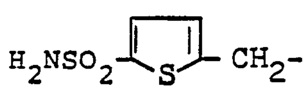 | H | 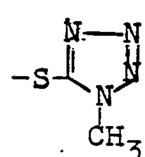 |
| 5 | 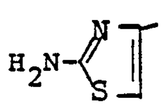 | 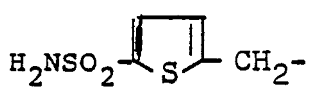 | $-OCH_3$ | 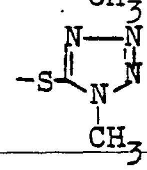 |

| | A | $R_2$ | Y | D |
|---|---|---|---|---|
| 6 | 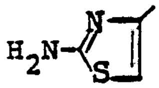 | 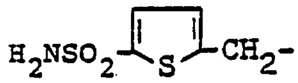 | H | 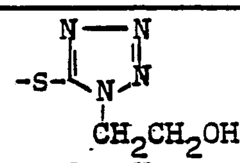 |
| 7 | 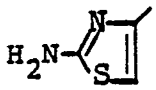 | 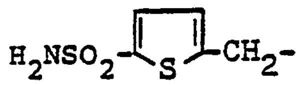 | $-OCH_3$ | 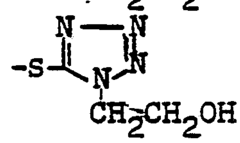 |
| 8 | 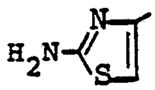 | 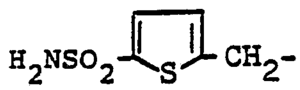 | H | 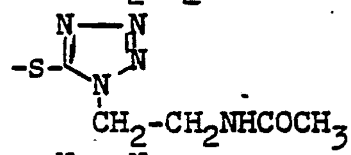 |
| 9 | 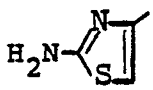 | 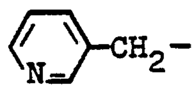 | H | 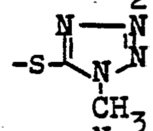 |
| 10 | 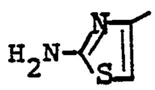 | 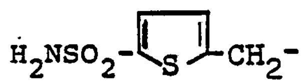 | H | 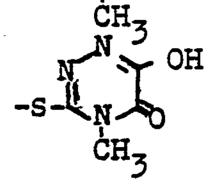 |
| 11 | 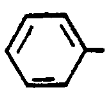 | 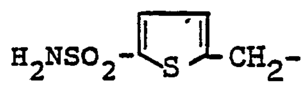 | $-OCH_3$ | 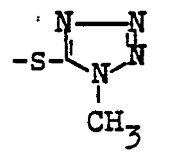 |
| 12 | 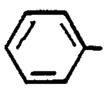 | 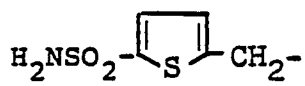 | $-OCH_3$ | $-OCOCH_3$ |
| 13 | 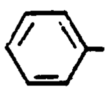 | 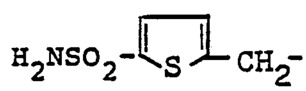 | $-OCH_3$ | 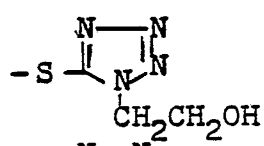 |
| 14 | 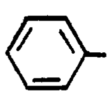 | 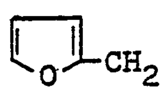 | $-OCH_3$ | 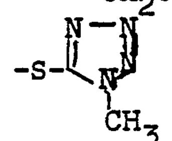 |
| 15 | 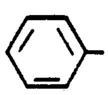 | 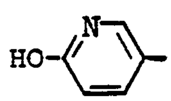 | $-OCH_3$ | 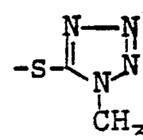 |
| 16 | 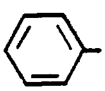 | 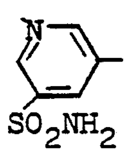 | $-OCH_3$ | 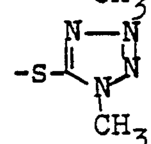 |
| 17 | 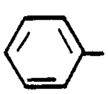 | 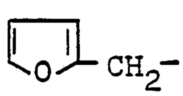 | $-OCH_3$ | 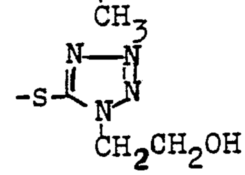 |

| | A | $R_2$ | Y | D |
|---|---|---|---|---|
| 18 | 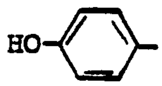 | 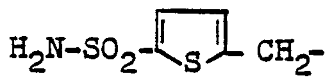 | $-OCH_3$ | 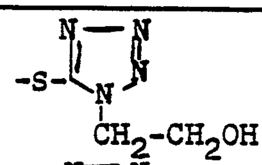 |
| 19 | 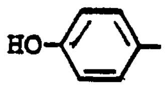 | 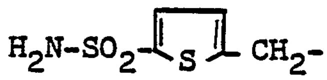 | $-OCH_3$ | 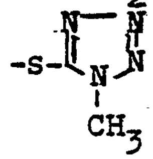 |
| 20 | 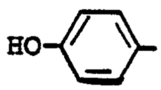 | 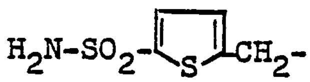 | $-OCH_3$ | $-OCOCH_3$ |
| 21 | 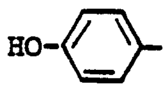 | 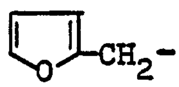 | $-OCH_3$ | $-OCOCH_3$ |
| 22 | 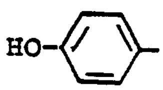 | 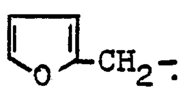 | $-OCH_3$ | 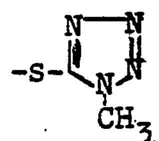 |
| 23 | 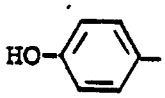 | 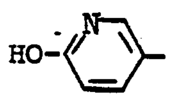 | $-OCH_3$ | 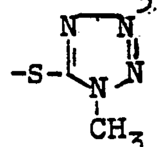 |
| 24 | 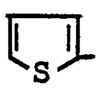 | 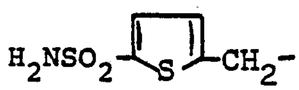 | $-OCH_3$ | 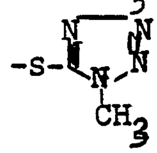 |

Die Wirksamkeit der erfindungsgemäßen ß-Lactam-Antibiotika läßt sich durch folgende Untersuchungen beispielhaft demonstrieren:

1. <u>In vitro-Versuche:</u>

Für die Untersuchungen wurde die Methode des Reihenverdünnungstestes im Mikrotitersystem angewandt. Die Prüfung der Substanzen auf Bakteriostase erfolgte in flüssigem Medium. Es wurde die Bakteriostasewirkung bei folgenden Konzentrationen untersucht:
128, 64, 32, 16, 8, 4, 2, 1, 0,5, 0,25, 0,12, 0,06 µg/ml.
Es wurde ein Nährboden folgender Zusammensetzung benutzt:
10 g Pepton, 8 g Fleischextrakt-Oxoid, 3 g Natriumchlorid, 2 g sek. Natriumphosphat werden mit dest. Wasser auf 100 ml aufgefüllt (pH 7,2 - 7,4). Das Alter der Primärkulturen betrug etwa 20 Stunden. Die Einstellung der Keimsuspension erfolgte am Photometer (nach "Eppendorf") (Reagenzglas-Durchmesser 14 mm, Filter 546 nm) anhand der Trübung einer Bariumsulfat-Vergleichssuspension, die durch eine Bariumsulfat-Aufschwemmung erzeugt wurde, welche durch die Zugabe von 3,0 ml 1 %ige Barium-chloridlösung in 97 ml 1 %ige Schwefelsäure entstand. Nach der Einstellung wurden die Testkeime im Verhältnis 1:1500 mit einer Kochsalzlösung weiter verdünnt.
16 mg der jeweiligen Substanz wurden in 10 ml-Meßkolben eingewogen und mit dem Lösungsmittel bis zur Marke aufgefüllt. Die weitere Verdünnungsreihe wurde mit destilliertem Wasser oder dem jeweiligen Lösungsmittel hergestellt.

Die Vertiefungen der Mikrotiterplatten wurden mit 0,2 ml Nährmedium, 0,01 ml der entsprechenden Substanzverdünnung und mit einem Tropfen Keimsuspension (0,01 ml) beschickt und 18 bis 20 Stunden bei 37°C bebrütet. Eine Lösungsmittelkontrolle wurde stets mitgeführt.

Die Ablesung wurde makroskopisch vorgenommen, wobei die jeweilige Grenzkonzentration (= niedrigste noch bakteriostatisch wirksame Konzentration) ermittelt wurde.

Als Testorganismen wurden benützt:

Staphylococcus aureus SG 511, Escherichia coli ATCC 11 775, Pseudomonas aeruginosa Hamburgensis und Pseudomonas aeruginosa BC 19, Serratia marcescens ATCC 13 880, Klebsiella pneumoniae ATCC 10 031 und BC 6, Proteus mirabilis BC 17, Proteus rettgeri, Enterobacter cloacae ATCC 13 047, E. coli R+TEM (ß-Lactamase-Träger), Klebsiella pneumoniae 1082 E (ß-Lactamase-Träger)

In der folgenden Tabelle 2 sind die ermittelten minimalen Hemmkonzentrationen (MIC) in µg/ml für typische Vertreter der erfindungsgemäßen Verbindungen aufgeführt, wobei die Numerierung derjenigen der Tabelle 1 entspricht.
Als Vergleichssubstanz diente Cefuroxim.

Tabelle 2

Minimale Hemmkonzentration in µg/ml

| Verbindungen der Tab. 1 | Staph. aureus SG 511 | E.coli ATCC 11775 | Pseud. Hbg. | Pseud. BC 19 | Serr. marcesc. ATCC 13880 | Kl.pneum. ATCC 10031 | Kl.pneum. BC 6 | Prot. mirab. BC 17 | Prot. rettg. | Ent. cloac. ATCC 13047 | E.coli R+TEM | K.pneum. 1082 E |
|---|---|---|---|---|---|---|---|---|---|---|---|---|
| 1 | 1 | 0,03 | 16 | 16 | 0,06 | 0,06 | 0,03 | 0,01 | 0,06 | 0,25 | 0,5 | 16 |
| 4 | 1 | 0,01 | 16 | 8 | 0,06 | 0,03 | 0,03 | 0,01 | 0,01 | 0,06 | 0,25 | 8 |
| 6 | 1 | < 0,03 | 16 | 8 | 0,06 | 0,06 | 0,03 | 0,03 | 0,06 | 0,06 | 0,5 | 16 |
| 11 | 1 | 0,12 | 16 | 8 | 0,25 | 0,25 | 0,25 | 0,06 | 0,5 | 0,5 | 0,25 | 0,25 |
| 14 | 0,5 | 0,12 | 8 | 8 | 0,25 | 0,25 | 0,25 | 0,12 | 1 | 1 | 0,25 | 0,25 |
| Cefuroxim | 1 | 8 | >128 | >128 | 8 | 2 | 4 | 0,5 | 2 | 32 | 4 | |

Die akute Toxizität wurde durch perorale und subcutane Applikation der Verbindungen der Tabelle 2 an der Ratte in steigenden Dosen bestimmt.

Die $LD_{50}$ ist die Dosis, nach deren Applikation 50 % der Tiere innerhalb von 8 Tagen sterben. Sämtliche Substanzen zeigten bei oraler Gabe eine $LD_{50}$ von über 4 g/kg, bei subcutaner Gabe eine $LD_{50}$ von über 2 g/kg, d. h., sie sind damit für die Praxis untoxisch.

Eine Reihe von erfindungsgemäßen Verbindungen wurde in vivo bei experimentellen Infektionen an Mäusen untersucht. Man verwendete als pathogene Bakterien E. coli ATCC 11 775. Es wurde eine intraperitoneale Infektion mit 0,2 ml einer Bakteriensuspension (mit 5 % Mucin) gesetzt. Dies entspricht etwa 1,4 x $10^6$ Keime E. coli/Maus Weibliche Mäuse vom Stamm NMRI wurden in Gruppen von jeweils 10 Tieren aufgeteilt, zwei Gruppen blieben unbehandelt, die restlichen Gruppen wurden mit verschiedenen Dosen der jeweiligen erfindungsgemäßen Penicilline und Cephalosporine subcutan zur Bestimmung der $ED_{50}$ (Dosis bei der 50 % der Tiere überleben) behandelt. Es wurde einmal eine Stunde nach Setzen der Injektion therapiert.

Der Beobachtungszeitraum betrug in beiden Fällen 7 Tage. Die Ergebnisse dieser Tests mit repräsentativen Vertretern der erfindungsgemäßen Cephalosporine sind in der Tabelle 3 dargestellt.

Tabelle 3

In vivo Aktivität bei Mäusen

E. coli-Infektion (s.c. Applikation):

| Verbindung der Tabelle 1 | $ED_{50}$ (mg/kg) |
|---|---|
| 1 | 0,8 |
| 4 | <0,5 |
| 6 | <0,5 |
| 11 | ~5 |
| 14 | 2,5 - 5 |
| Cefuroxim | >100 |

Es ist eine weitere Aufgabe der vorliegenden Erfindung, pharmazeutische Mittel zu schaffen, die bei der Behandlung infektiöser Krankheiten sowohl beim Menschen als auch beim Tier wertvoll sind.

Als bevorzugte pharmazeutische Zubereitungen seien Tabletten, Dragées, Kapseln, Granulate, Suppositorien, Lösungen, Suspensionen, Emulsionen, Salben, Gele, Cremes, Puder und Sprays genannt. Vorteilhafterweise wird der Wirkstoff in der Human- oder Tiermedizin oder ein Gemisch der verschiedenen Wirkstoffe der allgemeinen Formel I in einer Dosierung zwischen 5 und 500, vorzugsweise 10-200 mg/kg Körpergewicht je 24 Stunden verabreicht, gegebenenfalls in Form mehrerer Einzelgaben. Eine Einzelgabe enthält den oder die erfindungsgemäßen Wirkstoffe, vorzugsweise in Mengen von etwa 1 bis etwa 250, insbesondere 10 bis 60 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitpunkt bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Im Falle der Anwendung als Futterzusatzmittel können die neuen Verbindungen in den üblichen Konzentrationen und Zubereitungen zusammen mit dem Futter bzw. mit Futterzubereitungen oder mit dem Trinkwasser gegeben werden. Dadurch kann eine Infektion durch gramnegative oder grampositive Bakterien verhindert, gebessert und/oder geheilt werden und ebenso eine Förderung des Wachstums und eine Verbesserung der Verwertung des Futters erreicht werden.

Die folgenden Beispiele sollen die Erfinder näher erläutern:

## I. Synthese der Ureidocarbonsäuren der allgemeinen Formel IV: für A = Aminothiazol

1a) D,L-α-[3-(2-(5'-Sulfamoyl-2'-thienylmethylamino)-4-hydroxy-5-pyrimidinyl)-ureido]-(2,3-dihydro-2-imino-4-thiazolyl)-essigsäure

1,73 g D,L-α-Amino-(2,3-dihydro-2-imino-4-thiazolyl)-essigsäure (0,01 Mol) werden mit 10 ml 1 n Natronlauge in einem Gemisch aus 60 ml Tetrahydrofuran und 20 ml Wasser gelöst.

3,01 g 5-Amino-2-(5'-sulfamoyl-2'-thienylmethylamino)-4-hydroxy-pyrimidin (0,01 Mol) werden in 50 ml trockenem Tetrahydrofuran aufgeschlämmt und mit 6 ml Diäthylaminotrimethylsilan bis zur Lösung zum Rückfluß erhitzt. Man engt im Vakuum zur Trockne ein, löst wieder in 50 ml Tetrahydrofuran und tropft diese Lösung unter Eiskühlung zu einer Lösung von 1,05 g Phosgen in 20 ml trockenem Tetrahydrofuran. Man rührt 15 Minuten bei Eiskühlung nach und engt dann im Vakuum auf das halbe Volumen ein.

Diese Lösung wird bei Eiskühlung zu der oben hergestellten Lösung zugetropft; dabei wird der pH-Wert auf 8,0 gehalten. Man entfernt die Kühlung und rührt eine Stunde bei Raumtemperatur nach. Anschließend wird mit 30 ml Wasser verdünnt und das Tetrahydrofuran im Vakuum entfernt. Die wäßrige Phase wird zweimal mit Essigester ausgeschüttelt, hierauf wird die wäßrige Phase mit 2 n Salzsäure auf pH 3,8 gestellt. Der ausgefallene Niederschlag wird abgesaugt und im Vakuum getrocknet.
Ausbeute: 3,12 g (62 %);
IR-Spektrum: 3300 (breit), 1640-1650, 1530 (breites Signal), 1155 $cm^{-1}$;
NMR-Spektrum (DMSO + $CD_3OD$) Signale bei ppm: 4,65 (s,breit, 2H), 5,15 (s,1H), 6,5 (s,1H), 7,05 (d,1H), 7,45 (d,1H), 8,15 (s,1H).

Analog wurden die Ureidocarbonsäuren der folgenden Tabelle synthetisiert (A' = Wasserstoff):

| | $R_2$ | Ausbeute in % | NMR-Spektrum (DMSO + $CD_3OD$) Signale bei ppm: |
|---|---|---|---|
| 1b | 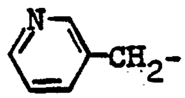 | 56 | 4,5(breit,2H), 5,15(s,1H), 6,45 (s,1H), 7,25(m,1H), 7,7 (m,1H), 8,1(s,1H), 8,5(m,2H). |
| 1c | 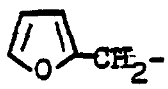 | 64,5 | 4,4(s,2H), 5,10(s,1H), 6,3(m,2H), 6,5(s,1H), 7,5(s,1H), 8,05(s,1H). |
| 1d | 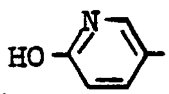 | 41 | 5,2(s,1H), 6,4(s,1H), 6,55(s,breit 1H), 7,55(q,1H), 7,8(d,1H), 8,2 (s,1H). |
| 1e | 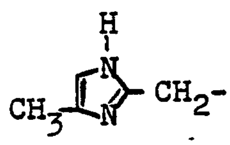 | 58 | 2,1(s,3H), 4,4(s,2H), 5,15(s,1H), 6,80(s,1H), 7,3(s,1H), 8,05(s,1H). |
| 1f | 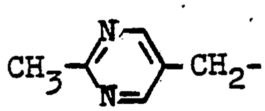 | 60 | 2,5(s,3H) 4;4(s,2H) 5,15(s,1H), 6,40(s,1H), 8,05(s,1H), 8,6(s,2H). |

## II. Synthese der Cephalosporine

### Beispiel 1

**Natrium-7ß-{D,L-α-[3-(4-hydroxy-2-(3'-pyridylmethylamino)-5-pyrimidinyl)-ureido]-2,3-dihydro-2-imino-4-thiazolyl)-acetamido}-3-[(1-methyl-tetrazol-5-yl)-thiomethyl]-ceph-3-em-4-carboxylat**

2,08 g (0,005 Mol) der Ureidocarbonsäure des Beispiels I/1b werden in 50 ml trockenem Dimethylformamid gelöst. Man gibt 2,5 g 7-Amino-3-[(1-methyl-tetrazol-5-yl)-thiomethyl]-ceph-3-em-4-carboxylat, gelöst in 30 ml Methylenchlorid zu und versetzt unter Eiskühlung mit 1,15 g Dicyclohexylcarbodiimid.

Man rührt über Nacht unter Eiskühlung, engt dann im Vakuum zur Trockne ein und rührt den Rückstand mit 50 ml Methanol und dann mit 100 ml Methylenchlorid aus. Das abgesaugte Festprodukt wird zur Entfernung geringfügiger Verunreinigungen über eine Silicagelsäule chromatographiert (Eluens Methylenchlorid/Methanol 5:1).

Der erhaltene Diphenylmethylester wird mit 10 ml Trifluoressigsäure und 4 ml Anisol in üblicher Weise gespalten und der Rückstand mit Natriumäthylhexanoat in Dimethylformamid/Methanol in das Natriumsalz überführt.
Ausbeute: 1,90 g (31 %);
IR-Spektrum: 3340, 1765, 1660, 1540 $cm^{-1}$;
NMR-Spektrum (DMSO + $CD_3OD$) Signale bei ppm: 3,50 (m,2H), 3,95(s,3H), 4,4(m,4H), 5,0(m,1H), 5,4(s,1H), 5,6(m.1H), 6,45 (d,1H), 7,25(m,1H), 7,65(m,1H), 8,1(s,1H), 8,5(m,2H).

Nach dieser Methode wurden die folgenden Cephalosporine der allgemeinen Formel I synthetisiert (A' = Wasserstoff, E = Natriumion, Y = Wasserstoff):

| | $R_2$ | D | Ausb. % | IR-Spek. $cm^{-1}$ | NMR-Spektrum (DMSO + $CD_3OD$) Sig. bei ppm: |
|---|---|---|---|---|---|
| 2 | 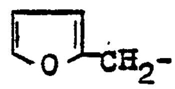 | 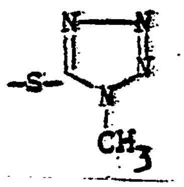 | 56 | 1765, 1660, 1540 | 3,50(m,2H), 3,95(s,3H), 4,4(m,4H), 5,05(m,1H), 5,40(s,1H), 5,65(m,1H), 6,3(m,2H), 6,45(d,1H), 7,5(s,1H), 8,05(s,1H). |
| 3 | 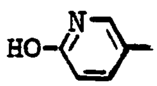 | 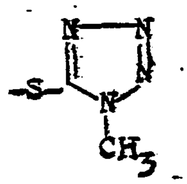 | 38 | 1765, 1655, 1545 | 3,55(m,2H), 3,95(s,3H), 4,4(m,2H), 5,05(m,1H), 5,35(s,1H), 5,60(m,1H), 6,45(s,1H), 6,55(s,1H, breit), 7,55(q,1H), 7,8(d,1H7, 8,2(s,1H). |
| 4 | 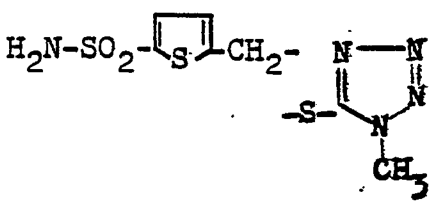 | | 61 | 1765, 1660, 1540, 1160 | 3,55(m,2H), 3,95(s,3H), 4,4(m,2H), 4,7(s,2H), 5,0(m,1H), 5,35(s,1H), 5,6(m,1H), 6,45(d,1H), 7,05(d,1H), 7,45(d,1H), 8,15(s,1H). |
| 5 | 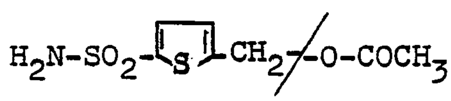 | | 48,5 | 1765, 1655, 1530, 1155 | 2,05(s,3H), 3,50(m,2H), 4,7(s,2H), 4,85-5,05 (m,2+1H), 5,40(s,1H), 5,65(m,1H), 6,45(breites s,1H), 7,05(d,1H), 7,45 (d,1H), 8,15(s,1H). |
| 6 | 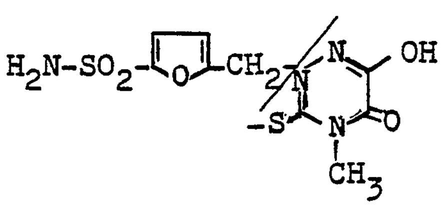 | | 49 | 1765 1700, 1670, (breit) | 3,3(s,3H), 3,60(m,2H), 4,2(m,2H), 4,65(s,2H), 5,0(d,1H), 5,40(s,1H), 5,65(m,1H), 6,45(s,1H), 7,0(d,1H), 7,45(d,1H), 8,15(s,1H). |

| | $R_2$ | D | Ausb. % | IR-Spek. $cm^{-1}$ | NMR-Spektrum (DMSO + $CD_3OD$) Sig. bei ppm: |
|---|---|---|---|---|---|
| 7 | 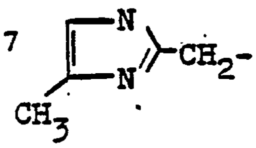 | 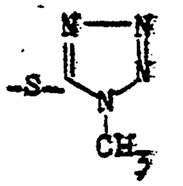 | 44 | 1765, 1650, 1520 | 2,1(s,3H), 3,50(m,2H), 3,95(s,3H), 4,4(m,4H), 5,05(breites d,1H), 5,40(s,1H), 5,60(m,1H), 6,80(s,1H), 7,3(s,1H), 8,05(s,1H). |
| 8 | 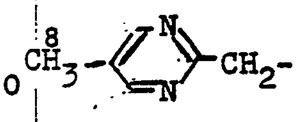 | 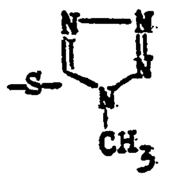 | 49,5 | 1765, 1655, 1540 | 2,45(s,3H), 3,45(m,2H), 3,95(s,3H), 4,3-4,5(m, 4H), 5,0(m,1H), 5,40 (s,1H), 5,65(m,1H), 6.45(breites s,1H), 8,10(s,1H), 8,55(s,2H). |

## Beispiel 9

**Natrium-7α-methoxy-7β-{D,L-α-[3-(2-(5'-sulfamoyl-2'-thienyl-methylamino)-4-hydroxy-5-pyrimidinyl)-ureido]-(2,3-dihydro-2-imino-4-thiazolyl)-acetamido}-3-[(1-methyl-tetrazol-5-yl)-thiomethyl]-ceph-3-em-4-carboxylat**

1,8 g (0,002 Mol) des Diphenylmethylesters des Cephalosporins des Beispiels 4 werden in 70 ml trockenem Tetrahydrofuran gelöst. Man gibt bei -70°C eine Lösung von 500 mg Lithiummethoxid in 20 ml trockenem Methanol zu und rührt bei dieser Temperatur 3 Minuten. Dann fügt man bei -70°C 300 mg t.-Butylhypochlorit zu. Man rührt 45 Minuten bei -70°C, gibt dann 0,6 ml Eisessig und 150 mg Triäthylphosphit zu. Bei Raumtemperatur wird mit 100 ml Phosphatpuffer (pH 7,0) versetzt und das Gemisch dreimal mit Methylenchlorid extrahiert. Die organische Phase wird abgetrennt, getrocknet und das Lösungsmittel im Vakuum entfernt. Der Rückstand wird zweimal über eine Silicagelsäule chromatographiert (Eluens Methanol/Methylenchlorid 1:6).

Man erhält 530 mg des gewünschten Diphenylmethylesters (30 % der Theorie).

Die Spaltung zur Säure und die Überführung in das Natriumsalz wird wie in Beispiel 1 durchgeführt.
IR-Spektrum: 1765, 1670, 1155 $cm^{-1}$;
NMR-Spektrum (DMSO + $CD_3OD$) Signale bei ppm: 3,40(s,3H), 3,55(m,2H), 3,95(s,3H), 4,35(m,2H), 4,7(s,2H), 5,05(breites s, 1H), 5,35(s,1H), 6,45(d,1H), 7,05(d,1H), 7,45(d,1H), 8,15 (s,1H).

Beispiel 10

Natrium-7α-methoxy-7β-{D,L-α-[3-(2-(5'sulfamoyl-2'-thienyl-methylamino)-4-hydroxy-5-pyrimidinyl)-ureido]-(2,3-dihydro-2-imino-4-thiazolyl)-acetamido}-3-[(1-(2'-hydroxyäthyl)-tetrazol-5-yl)-thiomethyl]-ceph-3-em-4-carboxylat

1,0 g (1,5 mMol) 7α-Methoxy-7β-{(D,L-α-amino)-2,3-(dihydro-2-imino-4-thiazolyl)-acetamido}-3-[(1-(2'-hydroxyäthyl)-tetrazol-5-yl)-thiomethyl]-ceph-3-em-4-carbonsäure-trifluoracetat werden in einem Gemisch von 60 ml Tetrahydrofuran und 20 ml Wasser gelöst. Der pH-Wert wird durch Zugabe von 1 n Natronlauge auf 8.5 gestellt.

450 mg 5-Amino-4-hydroxy-2-(5'-sulfamoyl-thienylmethylamino)-pyrimidin werden wie üblich silyliert und mit 150 mg Phosgen umgesetzt. Das entstehende Gemisch wird zu der Lösung des Cephalosporins gegeben, wobei der pH-Wert mit Natronlauge zwischen 7.5 und 8.0 gehalten wird. Nach Zugabe rührt man noch eine Stunde bei Raumtemperatur nach. Dann werden weitere 10 ml Wasser zugegeben, von etwas Unlöslichem abfiltriert und das Tetrahydrofuran im Vakuum abgezogen. Die wäßrige Phase wird zweimal mit Essigester ausgeschüttelt und dann unter Eiskühlung mit verdünnter Salzsäure auf pH 3,5 gestellt. Der Niederschlag wird abgesaugt, getrocknet und wie üblich in das Natriumsalz überführt.

Ausbeute: 655 mg (44,5 %)
IR-Spektrum: 1765, 1775 $cm^{-1}$;
NMR-Spektrum (DMSO + $CD_3OD$) Signale bei ppm: 3,43(s,3H), 3,7(m,2H), 4,3(m,2+2+2H), 4,65(s,2H), 5,1(s,breit,1H), 5,4(s,1H), 6,5(s,1H), 7,05(d,1H), 7.4(d,1H) 8,13(s,1H).

Analog Beispiel 9 werden folgende 7α-Methoxy-cephlosporine synthetisiert:

Beispiel 11

Natrium-7α-methoxy-7β-{D,L-α/3-(4-hydroxy-2-(3'pyridylmethyl-amino)-5-pyrimidinyl)-ureido7-(2,3-dihydro-2-imino-4-thiazolyl)-acetamido -3-/(1-methyl-tetrazol-5-yl)-thiomethyl7-ceph-3-em-4-carboxylat

Ausbeute: 36,5 % (der Theorie);
IR-Spektrum: 1765, 1660, 1540 $cm^{-1}$;
NMR-Spektrum (DMSO + $CD_3OD$) Signale bei ppm: 3,40(s,3H), 3,55(m,2H), 3,95(s,3H), 4,4(m,4H), 5,0(s,breit,1H), 5,35(s,1H), 6,45(s,breit,1H), 7,25(m,1H), 7,65(m,1H), 8,1(s,1H), 8,5(m,2H).

Beispiel 12

Natrium-7α-methoxy-7β-{D,L-α-/3-(4-hydroxy-2-(4'-methyl-2'-imidazolyl-methylamino)-5-pyrimidinyl)-ureido7-(2,3-dihydro-2-imino-4-thiazolyl)-acetamido}-2-/(1-methyl-tetrazol-5-yl)-thiomethyl7-ceph-3-em-4-carboxylat

Ausbeute: 34 %(der Theorie);
IR-Spektrum: 1765, 1655, 1535 $cm^{-1}$;
NMR-Spektrum (DMSO + $CD_3OD$) Signale bei ppm: 2,1(s,3H), 3,40 (s,3H), 3,50(m,2H), 3,95(s,3H), 4,4(m,4H), 5,0(s,1H), 5,35 (s,1H), 6,85(d,1H), 7,3(s,1H), 8,05(s,1H).

Beispiel 13

Natrium-7ß-{D,L-α-[3-(2-(5'-sulfamoyl-2'-thienylmethyl-amino)-4-hydroxy-5-pyrimidinyl)-ureido]-(2,3-dihydro-2-imino-4-thiazolyl)-acetamido}-3-[(1-(2'-hydroxyäthyl)-tetrazol-5-yl)-thiomethyl]-ceph-3-em-4-carboxylat

600 mg des Cephalosporins des Beispiel 5 werden zusammen mit 220 mg 1-(2'-Hydroxyäthyl)-5-mercapto-tetrazol in 40 ml Nitromethan 6 Stunden lang auf 80°C erwärmt. Man engt im Vakuum zur Trockne ein. Der Rückstand wird in einem Gemisch aus Aceton und Essigester gelöst. Unter Eiskühlung gibt man solange Diphenyldiazomethan zu, bis die Violettfärbung erhalten bleibt. Man engt danach zur Trockne ein. Der Rückstand wird durch Säulenchromatographie gereinigt (Silicagel, Eluens Methylenchlorid/Methanol 5:1). Der erhaltene Ester wird wie üblich gespalten und die Säure ins Natriumsalz überführt.
Ausbeute: 280 mg (43 %);
IR-Spektrum: 1765, 1660, 1540, 1155 $cm^{-1}$;
NMR-Spektrum (DMSO + $CD_3OD$) Signale bei ppm: 3,55(m,2H), 4,1-4,7(m,8H), 5,05(m,1H), 5,35(s,1H), 5,60(m,1H), 6,45(breites s, 1H), 7,05(d,1H), 7,45(d,1H), 8,15(s,1H).

Analog wurde hergestellt:

Beispiel 14

Natrium-7ß-{D,L-α-[3-(2-(5'-sulfamoyl-2'-thienylmethyl-amino)-4-hydroxy-5-pyrimidinyl)-ureido]-(2,3-dihydro-2-imino-4-thiazolyl)-acetamido}-3-[(2-methyl-1,3,4-thiadiazol-5-yl)-thiomethyl]-ceph-3-em-4-carboxylat

Ausbeute: 46,5 % (der Theorie);
IR-Spektrum: 1765, 1660, 1535, 1160 $cm^{-1}$;
NMR-Spektrum (DMSO + $CD_3OD$) Signale bei ppm: 2,75(s,3H), 3,50 (m,2H), 4,25(m,2H), 4,65(s,2H), 5,05(m,1H), 5,40(s,1H), 5,60 (m,1H), 6,45(d,1H), 7,05(d,1H), 7,45(d,1H), 8,10 (s,1H).

Beispiel 15

**Natrium-7ß-{D,L-α-[3-(2-(5'-sulfamoyl-2'-thienylmethylamino)-4-hydroxy-5-pyrimidinyl)-ureido]-(2,3-dihydro-2-imino-4-thiazolyl)-acetamido}-3-[(1-(2'acetylaminoäthyl)-tetrazol-5-yl)-thiomethyl]-ceph-3-em-4-carboxylat**

Ausbeute: 42 % der Theorie;
IR-Spektrum: 1760, 1655, 1610 $cm^{-1}$;
NMR-Spektrum: 1.85(s,3H), 3,6(m,2+2H), 4,2 - 4,7(m,6H), 5,05(q,1H), 5,40(s,breit,1H), 5,70(q,1H), 6,50(s,1H), 7,05(d,1H), 7,45(d,1H), 8,15(s,1H).

Beispiel 16

**Natrium-7α-methoxy-7ß-{D-α-[3-(2-(5'-sulfamoyl-2'-thienylmethylamino)-4-hydroxy-5-pyrimidinyl)-ureido]-phenylacetamido}-3-[(1-methyl-tetrazol-5-yl)-thiomethyl]-ceph-3-em-4-carboxylat**

1,9 g 7α-Methoxy-7ß-(D-α-amino-phenylacetamido)-3-[(1-methyltetrazol-5-yl)-thiomethyl]-ceph-3-em-4-carbonsäure (0,004 Mol) werden in einem Gemisch von 60 ml Tetrahydrofuran und 20 ml Wasser suspendiert. Unter Eiskühlung wird 1 n Natronlauge zugegeben, wobei Lösung eintritt.
Zu dieser Lösung wird ein, analog Beispiel 10, aus 1,2 g 5-Amino-4-hydroxy-2-(5-sulfamoyl-thienylmethylamino)-pyrimidin (0,004 Mol) hergestelltes Phosgen-addukt unter Eiskühlung zugetropft. Dabei wird der pH-Wert durch Zugabe von Natronlauge bei pH 8.0 gehalten.
Die weitere Umsetzung und Aufarbeitung erfolgt analog Beispiel 10.
Ausbeute: 1,53 g Natriumsalz (46 %)
IR-Spektrum: 1760, 1660 $cm^{-1}$
NMR-Spektrum (DMSO + $CD_3OD$) Signale bei ppm: 3,4 - 3,6(m,2H), 3,50(s,3H), 3,95(s,3H), 4,2(m,2H), 4,6(m,2H), 5,05(s,1H), 5,55(s,1H), 7,05(d,1H), 7,5(m,6H), 8,15(s,1H).

Analog Beispiel 16 wurden hergestellt (A = Phenyl, Y = Methoxy, E = Wasserstoff)

| | $R_2$ | D | Ausb. % | IR-Spek. $cm^{-1}$ | NMR-Spektrum (DMSO + $CD_3OD$) Sig. bei ppm: |
|---|---|---|---|---|---|
| 17 | 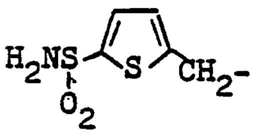 | $-OCOCH_3$ | 54,5 | 1765, 1670 | 2,05(s,3H), 3,5(s,3H +m,2H), 4,7(m,2+2H), 5,05(s,1H), 5,5(s, 1H), 7,05(d,1H), 7,55(m,5H+1H),8,13 (s,1H). |
| 18 | 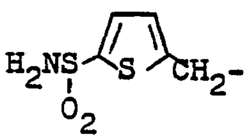 | 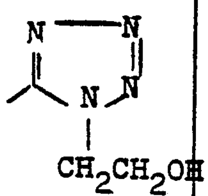 | 38 | 1765 | 3,48(s,3H), 3,6(m,2H) 4,3(m,6H), 4,6(m,2H) 5,0(s,1H), 5,55(s,1H) 7,05(d,1H), 7,5(m,6H) 8,15(s,1H), |
| 19 | 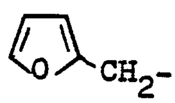 | 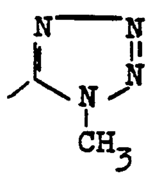 | 51 | 1765, 1670, 1610 | 3,50(s,3H), 3,5(m,2H) 3,95(s,3H), 4,2-4,4 (m,2H+2H), 5,0(s,1H), 5,55(s,1H), 6,3(m,2H) 7,5(m,6H), 8,15(s,1H) |
| 20 | 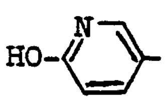 | 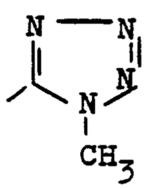 | 40 | 1765 1660 | 3,50(s,3H, m,2H), 3,95(s,3H), 4,25(m, 2H), 5,05(s,1H), 5,55(s,1H), 6,35(d, 1H), 7,5(m,6H), 7,8 (s,1H), 8,05(s,1H) |
| 21 | 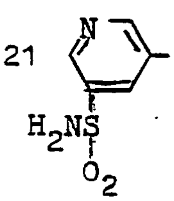 | 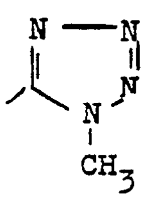 | 34 | 1765 | 3,50(s,3H+m,2H), 3,95(s,3H), 4,25 (m,2H), 5,05(s,1H), 5,55(s,1H), 7,5(m, 5H+1H), 8,05(s,1H), 8,2(m,2H) |

| | $R_2$ | D | Ausb. % | IR-Spek. $cm^{-1}$ | NMR-Spektrum (DMSO + $CD_3OD$) Sig. bei ppm: |
|---|---|---|---|---|---|
| 22 | 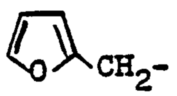 | 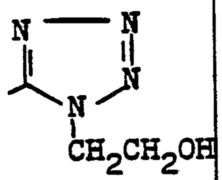 | 56 | 1765, 1660, 1610 | 3,48(s,3H), 3,4 - 3,6 (m,2H), 4,3(m,8H), 5,0(s,1H), 5,55(s,1H) 6,3(m,2H), 7,5(m,5+1H), 8,10(s,1H) |

**Beispiel 23**

**Natrium-7α-methoxy-7β-{D-α-[3-(4-hydroxy-2-(4'-methyl-2'-imidazolylmethylamino)-5-pyrimidinyl)-ureido]-p-hydroxyphenyl-acetamido}-3-[(1-methyl-tetrazol-5-yl)-thiomethyl]-ceph-3-em-4-carboxylat**

a) D-α-[3-(4-Hydroxy-2-(4'-methyl-2'-imidazolylmethylamino)-5-pyrimidinyl)-ureido]-p-hydroxyphenylessigsäure.

1,1 g (0,005 Mol) 5-Amino-4-hydroxy-2-(4'-methyl-2'-imidazolyl-methylamino)-pyrimidin werden in 60 ml trockenem Tetrahydrofuran mit 4 ml Diäthylaminotrimethylsilan bis zur Lösung am Rückfluß erhitzt. Die Lösung wird im Vakuum zur Trockne eingeengt und erneut in 50 ml Tetrahydrofuran aufgenommen. DieseLösung wird unter Eiskühlung zu einer Lösung von 525 mg Phosgen in Tetrahydrofuran getropft. Nach 15 Minuten wird im Vakuum auf das halbe Volumen eingeengt. Diese Lösung wird bei 5°C zu einer Lösung von 850 mg p-Hydroxy-phenylglycin in 40 ml Tetrahydrofuran, 10 ml Wasser und 5 ml 1n-Natronlauge getropft. Nach dem Ende der Zugabe wird der pH-Wert auf 8,0 gestellt und 1 Stunde bei Raumtemperatur nachgerührt. Anschließend wird mit 20 ml Wasser verdünnt, das Tetrahydrofuran im Vakuum abgezogen und zweimal mit Essigester ausgeschüttelt. Die wäßrige Phase wird unter Kühlung mit 2n-Salzsäure auf pH 2,9 gestellt, der Niederschlag abgesaugt und getrocknet.

Ausbeute: 1,45 g (70,5 % der Theorie);
IR-Spektrum: 3350, 1660, 1540, 1530 $cm^{-1}$;
NMR-Spektrum (DMSO + $CD_3OD$) Signale bei ppm: 2,1(s,3H), 4,4(s,2H), 5,15(s,1H), 6,75 (m,2H+1H), 7,3(d,2H), 8,15(s,1H)

b) 1,25 g (0,003 Mol) der so erhaltenen Ureidocarbonsäure werden in 40 ml trockenem Dimethylformamid gelöst. Man fügt zuerst 1,5 g 7-Amino-3-/(1-methyl-tetrazol-5-yl)-thiomethyl/-ceph-3-em-4-carbonsäurediphenylmethylester, gelöst in 20 ml Methylenchlorid zu und dann unter Eiskühlung 650 mg Dicyclohexylcarbodiimid. Man rührt über Nacht bei Raumtemperatur und engt anschließend im Vakuum zur Trockne ein. Der Rückstand wird zuerst mit 50 ml Methanol und dann mit 100 ml Methylenchlorid ausgerührt. Zur Entfernung geringfügiger Verunreinigungen chromatographiert man über eine Kieselgelsäule (Methylenchlorid/Methanol 5:1). Man erhält dabei den in 7α-Stellung nicht methoxylierten Diphenylmethylester der Titelverbindung.
Ausbeute: 1,62 g (62 % der Theorie).

c) 890 mg dieses Esters werden in 50 ml trockenem Tetrahydrofuran gelöst. Man gibt bei -70°C eine Lösung von 240 mg Lithiummethoxid in 10 ml Methanol zu und rührt 3 Minuten. Daraufhin wird bei -70°C 150 mg t-Butylhypochlorit zugegeben. Man rührt 45 Minuten bei -70°C und fügt dann 0,3 ml Eisessig und 75 mg Triäthylphosphit zu. Bei Raumtemperatur wird mit 70 ml Phosphatpuffer versetzt (pH 7,0) und das Gemisch dreimal mit Methylenchlorid extrahiert. Die organische Phase wird abgetrennt, getrocknet und das Lösungsmittel im Vakuum entfernt. Der Rückstand wird über eine Silicagelsäule chromatographiert (Eluens Methylenchlorid/Methanol 6:1). Man erhält 350 mg (38,5 % der Theorie) des Diphenylesters der Titelverbindung.

Dieser Ester wird 30 Minuten lang unter Eiskühlung mit 4 ml Trifluoressigsäure, 2 ml Anisol und 10 ml Methylenchlorid gerührt. Anschließend wird im Vakuum zur Trockne eingeengt und mit Äther versetzt. Das Festprodukt wird abgesaugt und in einem Dimethylformamid/Methanol Gemisch mit der entsprechenden Menge Natriumhexanoat zur Herstellung des Natriumsalzes behandelt. Dieses wird mit Äther ausgefällt, abgesaugt und im Vakuum getrocknet.
IR-Spektrum: 1765, 1660, 1550 $cm^{-1}$;
NMR-Spektrum (DMSO + $CD_3OD$) Signale bei ppm: 2,1(s,3H), 3,45(s,3H), 3,55(m,2H), 3,95(s,3H), 4,35(m,4H), 5,0(s,1H), 5,40(s,1H), 6,75(m,2+1H), 7,30(d,2H), 8,05(s,1H).

Analog den Beispielen 16 oder 23 lassen sich die Methoxycephalosporine der folgenden Tabelle herstellen. Die Synthese der entsprechenden Ureidocarbonsäuren sowie die Synthese der 7α-H-Benzhydrylester ist in der deutschen Offenlegungsschrift P 29 28 344 beschrieben.

| Beispiel | A | $R_2$ | D | Ausb. % | IR-Spektrum $cm^{-1}$ | NMR-Spektrum (DMSO + $CD_3OD$) Signale bei ppm: |
|---|---|---|---|---|---|---|
| 24 | 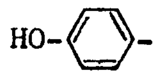 | 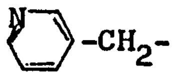 | 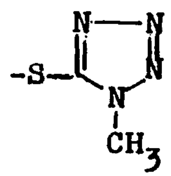 | 33,5 | 1765, 1660, 1540 | 3,45(s,3H), 3,55(m,2H), 3,95(s,3H), 4,25-4,45(m,4H), 4,95(s,1H), 5,35 (s,1H), 6,75(d,2H), 7,25(m,3H), 7,7 (m,1H), 8,1(s,1H), 8,5(m,2H). |
| 25 | 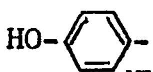 | 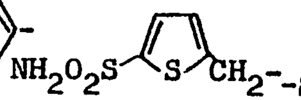 | 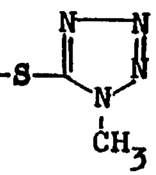 | 41 | 1765, 1655, 1535 | 3,45(s,3H), 3,55(m,2H), 3,95(s,3H), 4,3(m,2H), 4,65(s,2H), 5,0(s,1H), 6,75(d,2H), 7,05(d,1H), 7,3(d,2H), 7,45(d,1H), 8,15(s,1H). |
| 26 | 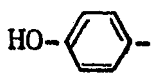 | 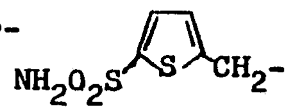 | $-OCOCH_3$ | 43 | 1765, 1660, 1540 | 2,05(s,3H), 3,45(s,3H), 3,45(m,2H), 4,7-5,0(m,2+2+1H), 5,35(s,1H), 6,75(d, 2H), 7,0-7,45(m,1+2+1H), 8,10(s,1H). |
| 27 | 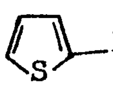 D,L | 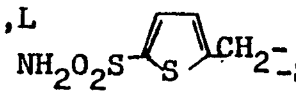 | 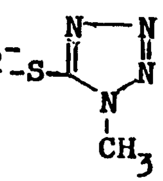 | 36 | 1765, 1655, 1540 | 3,45(s,3H), 3,55(m,2H), 3,95(s,3H), 4,3(m,2H), 4,7(s,2H), 5,0(s,1H), 5,70(s,1H), 7,0(m,3H), 7,4(m,2H), 8,1(s,1H). |
| 28 | 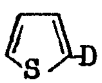 D,L | 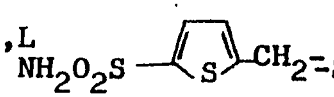 | 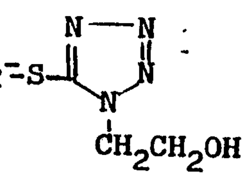 | 39 | 1765, 1665 1610, 1540 | 3,45(s,3H+m,2H), 4,3(m,6H), 4,65 (s,2H), 4,95(s,1H), 5,70(s,1H), 7,0(m,3H), 7,4(m,2H), 8,1(s,1H). |

| Beispiel | A | $R_2$ | D | Ausb. % | IR-Spektrum $cm^{-1}$ | NMR-Spektrum (DMSO + $CD_3OD$) Signale bei ppm: |
|---|---|---|---|---|---|---|
| 29 | 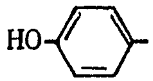 | 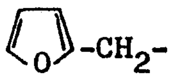 | 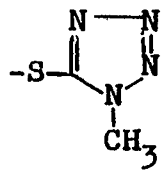 | 33 | 1765, 1660, 1550 | 3,45(s,3H), 3,50(m,2H), 3,95(s,3H), 4,40(m,4H), 4,95(s,1H), 5,35(s,1H), 6,3(m,2H), 6,75(d,2H), 7,25(d,2H), 7,45(s,1H), 8,1(s,1H). |
| 30 | 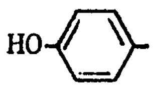 | 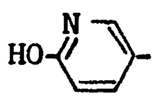 | 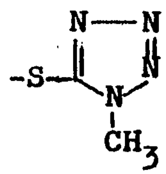 | 27 | 1765, 1655, 1540 | 3,45(s,3H), 3,50(m,2H), 3,95(s,3H), 4,3(m,2H), 4,95(s,1H), 5,35(s,1H), 6,55(s,breit,1H), 6,75(d,2H), 7,25 (d,2H), 7,55(m,1H), 7,8(d,1H), 8,15 (s,1H). |
| 31 | 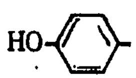 | 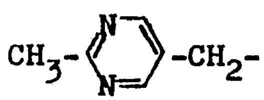 | 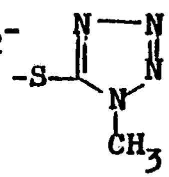 | 25 | 1765, 1660, 1550 | 2,50(s,3H), 3,45(s,3H), 3,55(m,2H), 3,95(s,3H), 4,4(m,4H), 4,95(s,1H), 5,40(s,1H), 6,75(d,2H), 7,25(d,2H), 8,05(s,1H), 8,6(s,2H). |

Beispiel 32

Natrium-7α-methoxy-7β-{D-α-[(2-(5'-aminosulfonyl-2'-thienylmethylamino)-4-hydroxy-5-pyrimidinyl)-ureido]-p-hydroxyphenylacetamido}-3-[(1-(2'-hydroxyäthyltetrazol-5-yl)-thiomethyl]-ceph-3-em-4-carboxylat

500 mg des Cephalosporins des Beispiels 26 werden zusammen mit 180 mg 1-(2'-Hydroxyäthyl)-5-mercapto-tetrazol in 40 ml Nitromethan 6 Stunden lang auf 80°C erwärmt. Man engt im Vakuum zur Trockne ein. Der Rückstand wird in einem Gemisch aus Aceton und Essigester gelöst. Unter Eiskühlung gibt man solange Diphenyldiazomethan zu, bis die Violettfärbung erhalten bleibt.

Man engt danach zur Trockne ein. Der Rückstand wird durch Säulenchromatographie gereinigt (Silicagel, Eluens Methylenchlorid/Methanol 5:1). Der erhaltene Ester wird, wie im Beispiel 1 beschrieben, gespalten und die Säure in ihr Natriumsalz überführt.

Ausbeute an Natriumsalz: 230 mg;

IR-Spektrum: 1765, 1660, 1540, 1155 $cm^{-1}$;

NMR-Spektrum (DMSO + $CD_3OD$) Signale bei ppm: 3,45(s,3H), 3,55(m,2H), 4,0-4,7(m,8H), 5,0(s,1H), 5,45(s,1H), 6,75(d,2H), 7,0(d,1H), 7,25(d,2H), 7,45(d,1H), 8,15(s,1H).

Analog wurde hergestellt:

Beispiel 33

Natrium-7α-methoxy-7β-{D,L-α-[3-(2-(5'-aminosulfonyl-2'-thienylmethylamino)-4-hydroxy-5-pyrimidinyl)-ureido]-p-hydroxyphenylacetamido}-3-[(2-methyl-1,3,4-thiadiazol-5-yl)-thiomethyl]-ceph-3-em-4-carboxylat

mit dem Cephalosporin des Beispiel 26 und 2-Methyl-5-mercapto-1,3,4-thiadiazol.
Ausbeute an Natriumsalz: 44 % der Theorie;
IR-Spektrum: 1765, 1655, 1610, 1540 $cm^{-1}$;
NMR-Spektrum (DMSO + $CD_3OD$) Signale bei ppm: 2,75(s,3H), 3,45(s,3H), 3,50(m,2H), 4,25(m,2H), 4,65(s,2H), 4,95(s,1H), 5,45(s,1H), 6,75(d,2H), 7,05(d,1H), 7,25(d,2H), 7,45(d,1H), 8,15(s,1H).

Die Verbindungen der allgemeinen Formel I und I' lassen sich in die üblichen pharmazeutischen Anwendungsformen, wie Tabletten, Dragées, Kapseln oder Ampullen einarbeiten. Die Einzeldosis beträgt bei Erwachsenen im allgemeinen zwischen 50 und 1000 mg, vorzugsweise 100 bis 500 mg, die Tagesdosis zwischen 100 und 4000 mg, vorzugsweise 250 bis 2000 mg.

## Beispiel I

**Tabletten enthaltend Natrium-7α-methoxy-7β-{D-α-[3-(4-hydroxy-2-(4'-methyl-2'-imidazolylmethylamino)-5-pyrimidinyl)-ureido]-p-hydroxyphenyl-acetamido}-3-[(1-methyl-tetrazol-5-yl)-thiomethyl]-ceph-3-em-4-carboxylat**

Ein Gemisch bestehend aus 2 kg Wirksubstanz, 5 kg Lactose, 1,8 kg Kartoffelstärke, 0,1 kg Magnesiumstearat und 0,1 kg Talk wird in üblicher Weise zu Tabletten gepreßt, derart, daß jede Tablette 200 mg Wirkstoff enthält.

**Beispiel II**

**Dragées enthaltend Natrium-7α-methoxy-7β-{D-α-[3-(4-hydroxy-2-(4'-methyl-2'-imidazolylmethylamino)-5-pyrimidinyl)-ureido]-p-hydroxyphenyl-acetamido}-3-[(1-methyl-tetrazol-5-yl)-thiomethyl]-ceph-3-em-4-carboxylat**

Analog Beispiel I werden Tabletten gepreßt, die anschließend in üblicher Weise mit einem Überzug bestehend aus Zucker, Kartoffelstärke, Talk und Tragant überzogen werden.

**Beispiel III**

**Kapseln enthaltend Natrium-7α-methoxy-7β-{D-α-[3-(4-hydroxy-2-(4'-methyl-2'-imidazolylmethylamino)-5-pyrimidinyl)-ureido]-p-hydroxyphenyl-acetamido}-3-[(1-methyl-tetrazol-5-yl)-thiomethyl]-ceph-3-em-4-carboxylat**

5 kg Wirksubstanz werden in üblicher Weise in Hartgelatinekapseln gefüllt, derart, daß jede Kapsel 500 mg des Wirkstoffes enthält.

**Beispiel IV**

**Trockenampullen enthaltend Natrium-7α-methoxy-7β-{D-α-[3-(4-hydroxy-2-(4'-methyl-2'-imidazolylmethylamino)-5-pyrimidinyl)-ureido]-p-hydroxyphenyl-acetamido}-3-[(1-methyl-tetrazol-5-yl)-thiomethyl]-ceph-3-em-4-carboxylat**

In einem aseptischen Bereich wurde 251 g Wirkstoff in 200 ml destilliertem Wasser zur Injektion aufgelöst. Die Lösung wurde durch ein Millipore-Filter (Porengröße 0,22 µm, Produkt der Millipore Corporation, Bedford, USA) filtriert. Die Lösung

wurde jeweils in einer Menge von 2,0 ml in 1000 Gläschen (Kapazität 10 ml) eingegossen und es wurde lyophilisiert. Die Gläschen wurden sodann mit einem Kautschukstöpsel und einer Aluminiumkappe verschlossen. Somit wurden Gläschen (Nr. A) jeweils mit 250 mg Wirkstoff erhalten.

Eine physiologische Kochsalzlösung zur Injektion wurde in einer Menge von jeweils 2,0 ml in Ampullen abgefüllt und die Ampullen wurden verschlossen. Auf diese Weise wurden Ampullen (Nr. B) erhalten. Die physiologische Kochsalzlösung in den Ampullen (Nr. B) wurde in die Gläschen (Nr. A) gegossen, wodurch eine injizierbare Zubereitung für die intravenöse Verabreichung erhalten wurde.

Destilliertes Wasser zur Injektion wurde in einer Menge von 20 ml in die Gläschen (Nr. A) gegossen und die Lösung wurde in einer 5%igen Lösung von Glucose für Injektionen (250 ml) aufgelöst. Auf diese Weise wurden Lösungen für die kontinuierliche Infusion hergestellt.

Analog sind Tabletten, Dragées, Kapseln und Ampullen erhältlich, die einen oder mehrere der übrigen Wirkstoffe der Formel I oder die physiologisch unbedenklichen Salze dieser Verbindungen enthalten.

# Patentansprüche

1. Neue Cephalosporine der allgemeinen Formel

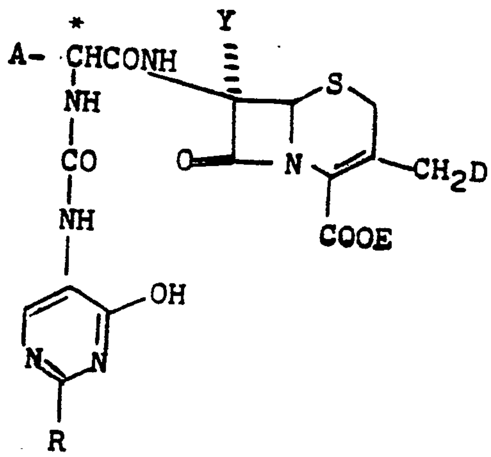

,(I)

bzw.

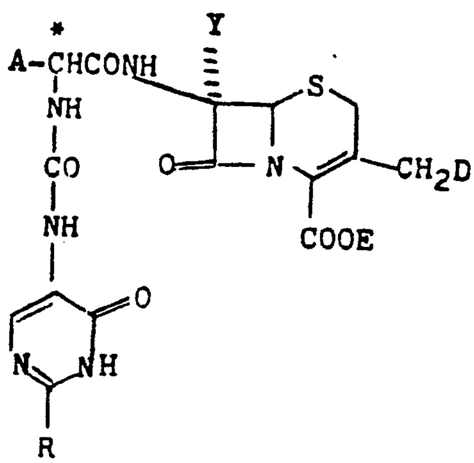

I'

in welchen die Reste A, Y, D, E und R folgende Bedeutungen besitzen:

Y ein Wasserstoffatom oder die Methoxygruppe

A sofern Y die Methoxygruppe darstellt, die Phenyl-, 4-Hydroxyphenyl-, 3,4-Dihydroxyphenyl- oder die 2- oder 3-Thienylgruppe, sofern Y ein Wasserstoffatom oder die Methoxygruppe darstellt, eine Gruppe der Formel

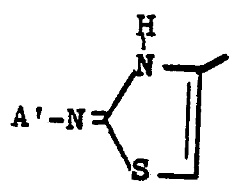

wobei in dieser Gruppe
A' ein Wasserstoffatom, die Gruppe $-COCH_2Cl$, $-COCH_2Br$, $-COOCH_2CCl_3$, die Formyl- oder die Tritylgruppe bedeutet,

D ein Wasserstoffatom, die Acetoxy- oder Aminocarbonyloxygruppe, die Pyridinium- oder 4-Aminocarbonylpyridiniumgruppe, oder die Gruppe -SHet, in welcher Het die 1,3,4-Thiadiazol-5-yl-, 2-Methyl-1,3,4-thiadiazol-5-yl-, die 1,2,4-Thiadiazol-5-yl-, 3-Methyl-1,2,4-thiadiazol-5-yl-, 4H-5,6-Dioxo-1,2,4-triazin-3-yl-, 4-Methyl-5,6-dioxo-1,2,4-triazin-3-yl-, 1-Vinyl-tetrazol-5-yl- oder 1-Allyl-tetrazol-5-ylgruppe oder eine Gruppe der allgemeinen Formel

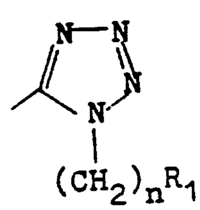

,(II)

bedeutet, in der n die Zahlen 1 bis 3 darstellen und $R_1$ für die Hydroxygruppe, die Amino-, Dimethylamino-, Acetylamino-, Aminocarbonyl-, Aminocarbonylamino-, Aminosulfonyl-, Aminosulfonylamino-, Methylcarbonyl-, Methylsulfonylamino-, Cyano-, Hydroxysulfonylamino-, Methylsulfonyl-, Methylsulfinyl-, sowie für eine Carbonsäure- oder Sulfonsäuregruppe steht, oder in der $(CH_2)_nR_1$ eine Alkylgruppe mit 1 bis 4 Kohlenstoffatomen oder einen 2,3-Dihydroxypropylrest bedeuten,

R eine Gruppe der allgemeinen Formel $-NHR_2$, in der $R_2$ eine substituierte oder unsubstituierte 3-Pyridyl-, 5-Pyrimidinyl-, 2-Thienyl-, 2-Furylmethyl-, 2-Thienylmethyl-, 2-Imidazolylmethyl-, 2-Thiazolylmethyl-, 3-Pyridylmethyl- oder eine 5-Pyrimidinylmethylgruppe darstellt, wobei diese Gruppen durch ein Chloratom, eine Methyl-, Acetylamino-, Hydroxy-, Methylsulfinyl-, Methylsulfonyl-, Aminocarbonyl- oder Aminosulfonylgruppe substituiert sein können, und

E ein Wasserstoffatom oder eine in vitro oder in vivo leicht abspaltbare Carboxylschutzgruppe

und, falls E ein Wasserstoffatom ist, ihre pharmakologisch verträglichen Salze mit anorganischen oder organischen Basen.

2. Neue Cephalosporine der allgemeinen Formel I bzw. I' gemäß Anspruch 1, dadurch gekennzeichnet, daß A die im Anspruch 1 genannten Bedeutungen hat, wobei A' ein Wasserstoffatom darstellt, Y, E und R wie im Anspruch 1 genannt definiert sind und D die Acetoxygruppe oder die Gruppe -SHet bedeutet, in der Het die 2-Methyl-1,3,4-thiadiazol-5-yl- oder 4-Methyl-5,6-dioxo-1,2,4-triazin-3-ylgruppe oder eine Tetrazol-5-yl-Gruppe der allgemeinen Formel II bedeutet, in der n und $R_1$ wie im Anspruch 1 genannt definiert sind, und ihre pharmakologisch verträglichen Salze mit anorganischen oder organischen Basen, falls E ein Wasserstoffatom bedeutet.

3. 7ß-{D,L-α-[3-(4-Hydroxy-2-(2'-furylmethylamino)-5-pyrimidinyl)-ureido]-(2,3-dihydro-2-imino-4-thiazolyl)-acetamido}-3-[(1-methyl-tetrazol-5-yl)-thiomethyl]-ceph-3-em-4-carbonsäure,

7ß-{D,L-α-[3-(2-(5'-aminosulfonyl-2'-thienylmethylamino)-4-hydroxy-5-pyrimidinyl)-ureido]-(2,3-dihydro-2-imino-4-thiazolyl)-acetamido}-3-[(1-methyl-tetrazol-5-yl)-thiomethyl]-ceph-3-em-4-carbonsäure,

7ß-{D,L-α-[3-(2-(5'-aminosulfonyl-2'-thienylmethylamino)-4-hydroxy-5-pyrimidinyl)-ureido]-(2,3-dihydro-2-imino-4-thiazolyl)-acetamido}-3-[(1-(2'-hydroxyäthyl)-tetrazol-5-yl)-thiomethyl]-ceph-3-em-4-carbonsäure,

7α-Methoxy-7ß-{D,L-α-[3-(2-(5'-aminosulfonyl-2'-thienylmethylamino)-4-hydroxy-5-pyrimidinyl-ureido]-phenylacetamido}-3-[(1-methyl-tetrazol-5-yl)-thiomethyl]-ceph-3-em-4-carbonsäure

und

7α-Methoxy-7ß-{D,L-α-[3-(2'-furylmethylamino)-5-pyrimidinyl)-ureido]-phenylacetamido}-3-[(1-methyl-tetrazol-5-yl)-thiomethyl]-ceph-3-em-4-carbonsäure

und deren physiologisch verträglichen Salze mit anorganischen oder organischen Basen.

4. Neue Cephalosporine der allgemeinen Formel I bzw. I' gemäß Anspruch 1, dadurch gekennzeichnet, daß E die Benzyl-, Diphenylmethyl-, Trityl-, t-Butyl-, 2,2,2-Trichloräthyl- oder Trimethylsilylgruppe, eine Alkanoyloxyalkylgruppe mit 3 bis 8 Kohlenstoffatomen oder die Phthalidylgruppe bedeutet.

5. Arzneimittel, gekennzeichnet durch einen Gehalt an einem oder mehreren Wirkstoffen der allgemeinen Formel I, bzw. I' gemäß den Ansprüchen 1 bis 4 neben den üblichen Träger- und/oder Hilfsstoffen.

6. Verfahren zur Herstellung neuer Cephalosporine der allgemeinen Formel

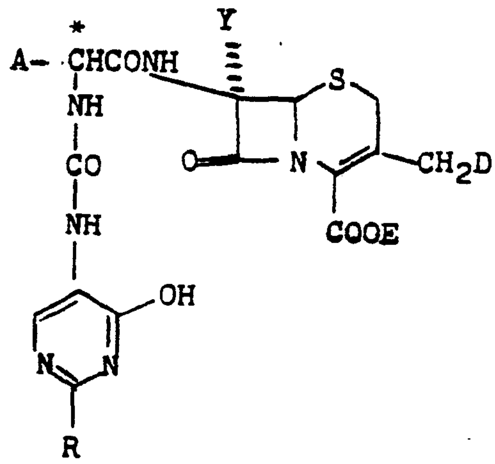

,(I)

bzw.

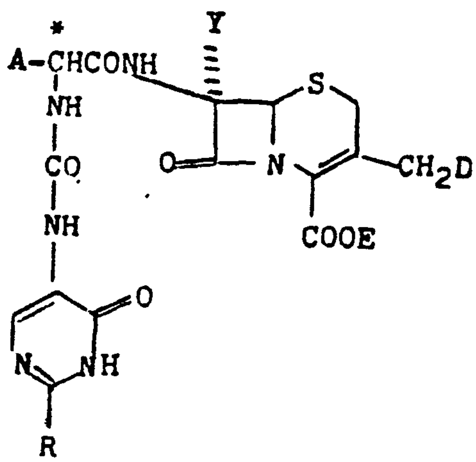

I'

in welchen die Reste A, Y, D, E und R die folgenden Bedeutungen besitzen:

Y ein Wasserstoffatom oder die Methoxygruppe

A sofern Y die Methoxygruppe darstellt,
die Phenyl-, 4-Hydroxyphenyl-, 3,4-Dihydroxyphenyl- oder die 2- oder 3-Thienylgruppe,
sofern Y ein Wasserstoffatom oder die Methoxygruppe darstellt,
eine Gruppe der Formel

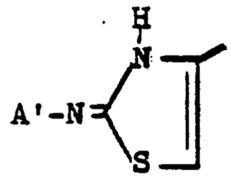

wobei in dieser Gruppe
A' ein Wasserstoffatom, die Gruppe $-COCH_2Cl$, $-COCH_2Br$, $-COOCH_2CCl_3$, die Formyl- oder die Tritylgruppe bedeutet,

D ein Wasserstoffatom, die Acetoxy- oder Aminocarbonyloxygruppe, die Pyridinium- oder 4-Aminocarbonylpyridiniumgruppe, oder die Gruppe -SHet, in welcher Het die 1,3,4-Thiadiazol-5-yl-, 2-Methyl-1,3-4-thiadiazol-5-yl-, die 1,2,4-Thiadiazol-5-yl-, 3-Methyl-1,2,4-thiadiazol-5-yl-, 4H-5,6-Dioxo-1,2,4-triazin-3-yl-, 4-Methyl-5,6-dioxo-1,2,4-triazin-3-yl-, 1-Vinyl-tetrazol-5-yl- oder 1-Allyl-tetrazol-5-ylgruppe oder eine Gruppe der allgemeinen Formel

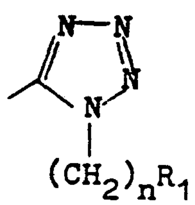 ,(II)

bedeutet, in der n die Zahlen 1 bis 3 darstellen und $R_1$ für die Hydroxygruppe, die Amino-, Dimethylamino-, Acetylamino-, Aminocarbonyl-, Aminocarbonylamino-, Aminosulfonyl-, Aminosulfonylamino-, Methylcarbonyl-, Methylsulfonylamino-, Cyano-, Hydroxysulfonylamino-, Methylsulfonyl-, Methylsulfinyl-, sowie für eine Carbonsäure- oder Sulfonsäuregruppe steht, oder in der $(CH_2)_nR_1$ eine Alkylgruppe mit 1 bis 4 Kohlenstoffatomen oder einen 2,3-Dihydroxypropylrest bedeutet,

R eine Gruppe der allgemeinen Formel $-NHR_2$, in der $R_2$ eine substituierte oder unsubstituierte 3-Pyridyl-, 5-Pyrimidinyl-, 2-Thienyl-, 2-Furylmethyl-, 2-Thienylmethyl-, 2-Imidazolylmethyl-, 2-Thiazolylmethyl-, 3-Pyridylmethyl- oder eine 5-Pyrimidinylmethylgruppe darstellt, wobei diese Gruppen durch ein Chloratom, eine Methyl-, Acetylamino-, Hydroxy-, Methylsulfinyl-, Methylsulfonyl-, Aminocarbonyl- oder Aminosulfonylgruppe substituiert sein können,

E ein Wasserstoffatom oder eine in vitro oder in vivo leicht abspaltbare Carboxylschutzgruppe,

desweiteren, sofern D für die Pyridinium- oder Aminocarbonylpyridiniumgruppe steht, von Verbindung an der allgemeinen Formel

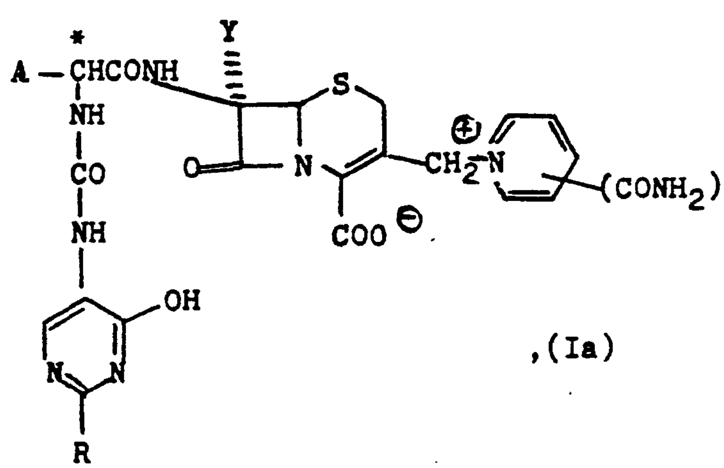

,(Ia)

in der A, R und Y wie oben definiert sind, und, falls E für Wasserstoff steht, von deren Salzen mit anorganischen oder organischen Basen, dadurch gekennzeichnet, daß

a) Zur Herstellung von Verbindungen der allgemeinen Formel I bzw. I', in der D die oben angegebenen Bedeutungen mit Ausnahme der einer Pyridinium- oder Aminocarbonylpyridiniumgruppe besitzt, ein 7-Aminocephalosporansäurederivat der allgemeinen Formel III

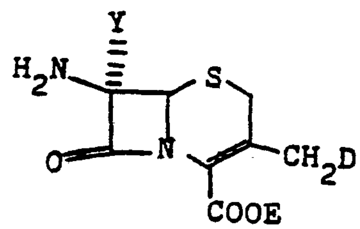

,(III)

in der Y und E wie oben definiert sind und D die obengenannten Bedeutungen mit Ausnahme der einer Pyridinium- oder Aminocarbonylpyridiniumgruppe aufweist, mit einer Ureidocarbonsäure der allgemeinen Formel IV

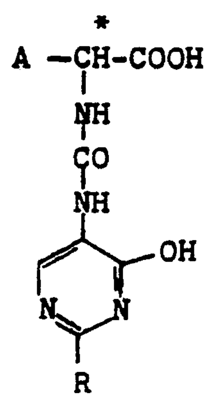

,(IV)

in der A und R die oben genannten Bedeutungen besitzen, oder mit deren Salzen oder reaktiven Derivaten bei Temperaturen zwischen -40°C und +40°C in Gegenwart eines Lösungsmittels und, gegebenenfalls, in Gegenwart einer Base umgesetzt wird, oder

b) eine 7-Aminocephalosporansäure der allgemeinen Formel V

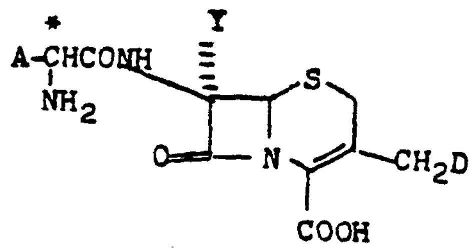

,(V)

in der A, D und Y wie oben definiert sind, oder ihre Salze mit anorganischen oder organischen Basen mit einem Pyrimidinderivat der allgemeinen Formel VI

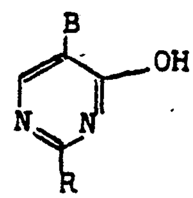

,(VI)

in der R wie oben definiert ist und B die Gruppe -NCO oder ein reaktives Derivat der Gruppe -NHCOOH bzw. die Gruppen -NHCOCl, -NHCOBr oder -NH-COO-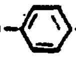-$NO_2$ bedeutet oder mit Gemischen solcher Pyrimidine der allgemeinen Formel VI, in der B teils die eine und teils die andere der vorstehend genannten Bedeutungen besitzt, oder in der diese Verbindungen auch teilweise oder ganz als ein den Isocyanaten isomeres 1-(H)-oxazolo(5,4-d)pyrimidin-2-on vorliegen können, in einem Lösungsmittel und einem pH-Bereich zwischen 2,0 und 9,0 bei Temperaturen zwischen -20°C und +50°C umgesetzt wird, oder

c) zur Herstellung von Verbindungen der allgemeinen Formel I bzw. I', in der D entweder die Gruppe -SHet mit den oben für Het angegebenen Bedeutungen oder die Pyridinium- oder Aminocarbonylpyridiniumgruppe bedeutet, und E für Wasserstoff steht, eine Verbindung der allgemeinen Formel VII

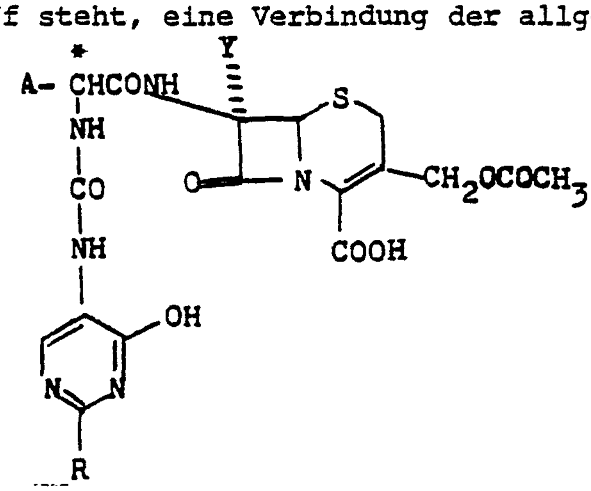 ,(VII)

in der A, Y und R wie oben definiert sind, entweder mit einer Verbindung der allgemeinen Formel VIII

Het-S-M ,(VIII)

in der Het die oben angegebenen Bedeutungen hat und M für ein Wasserstoffatom oder ein Alkalimetall oder ein Erdalkalimetall steht oder mit Pyridin bzw. 4-Aminocarbonylpyridin in einem Lösungsmittel bei Temperaturen zwischen $0^{o}$ und $100^{o}C$ in einem pH-Bereich zwischen 2 und 10 zu einer Verbindung der allgemeinen Formel I bzw. I', in der D die Bedeutung einer Gruppe -SHet oder einer Pyridinium- oder 4-Aminocarbonylpyridiniumgruppe und E die eines Wasserstoffatoms besitzt, umgesetzt wird, oder

d) Zur Herstellung einer Verbindung der allgemeinen Formel I bzw. I', in der Y die Methoxygruppe bedeutet, eine Verbindung der allgemeinen Formel IX

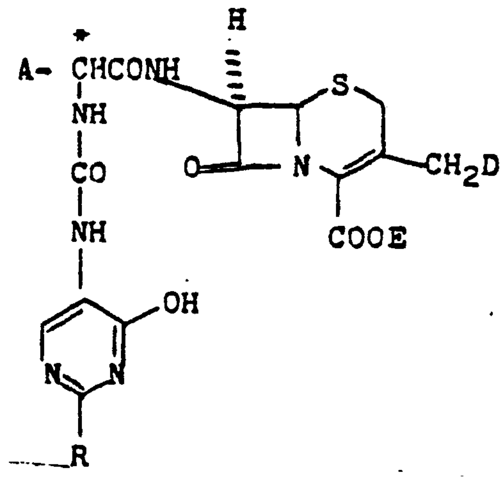

,(IX)

in der A, R, D und E wie oben definiert sind, in Anwesenheit von Methanol mit einem Alkalimetall-Methylat der allgemeinen Formel $M^+OCH_3^-$, worin $M^+$ ein Alkalimetall bedeutet, und dann mit einem Halogenierungsmittel in einem inerten Lösungsmittel bei Temperaturen zwischen $-120^\circ$ und $-10^\circ C$ umgesetzt wird und,
gewünschtenfalls anschließend, eine so erhaltene Verbindung der allgemeinen Formel I bzw. I', worin E eine in vitro oder in vivo leicht abspaltbare Schutzgruppe darstellt, in die freie Carbonsäure der allgemeinen Formel I bzw. I', in der E ein Wasserstoffatom ist, überführt wird und/oder eine Verbindung der allgemeinen Formel I bzw. I', in der E ein Wasserstoffatom ist, in ihre Ester oder, mittels anorganischer oder organischer Basen, in die entsprechenden Salze übergeführt wird.

7. Verfahren gemäß Anspruch 6a, dadurch gekennzeichnet, daß man als reaktive Derivate der Ureidocarbonsäuren der allgemeinen Formel IV deren Säureanhydride, deren reaktive Ester, deren reaktive Amide, deren Säurehalogenide oder Säureazide und als 7-Aminocephalosporansäurederivate der allgemeinen Formel III solche Verbindungen verwendet, bei welchen E die eingangs erwähnten Bedeutungen mit Ausnahme der eines Wasserstoffs besitzt, und die Umsetzung in einem Lösungsmittel,

gegebenenfalls in Anwesenheit einer Base und/oder eines Kondensationsmittels erfolgt.

8. Verfahren gemäß Anspruch 6b, dadurch gekennzeichnet, daß eine Verbindung der allgemeinen Formel V oder eines ihrer Salze mit anorganischen oder organischen Basen mit einer Verbindung der allgemeinen Formel VI,

   a) in Wasser oder in mit Wasser mischbaren Lösungsmitteln in Gegenwart von Wasser in einem pH-Bereich von 6,5 bis 8,0 oder
   b) in wasserfreien Lösungsmitteln oder
   c) in einem Gemenge aus Wasser und mit Wasser nicht mischbaren Lösungsmitteln in einem pH-Bereich zwischen 6,5 und 8,0

   umgesetzt wird, oder daß eine Verbindung der allgemeinen Formel V, in der das Wasserstoffatom der Carboxylgruppe durch eine Silylgruppe oder eine andere leicht abspaltbare Schutzgruppe ersetzt ist, mit einer Verbindung der allgemeinen Formel VI in einem wasser- und hydroxylgruppenfreien bzw. aprotischen Lösungsmittel, gegebenenfalls in Gegenwart einer Base, umgesetzt wird.

9. Verfahren gemäß Anspruch 6c, dadurch gekennzeichnet, daß die Umsetzung in einem stark polaren Lösungsmittel bei einem pH-Wert von 4 bis 8 durchgeführt wird.

10. Verfahren gemäß Anspruch 6d, dadurch gekennzeichnet, daß eine Verbindung der allgemeinen Formel IX in einem inerten Mittel gelöst oder suspendiert, mit 2 bis 6 Äquivalenten eines Alkalimetallmethylats in überschüssigem Methanol zusammengebracht und anschließend mit einem Halogenierungsmittel bei Temperaturen zwischen -100 und -50°C umgesetzt unß die Reaktion nach erfolgter Halogenierung durch Säurezusatz abgebrochen wird.

11. Verfahren gemäß Anspruch 6, dadurch gekennzeichnet, daß ein Alkalisalz der Cephalosporansäure der allgemeinen Formeln I oder I' mit einem Pivaloyloxymethylhalogenid oder Halogenmethylacetat, Halogenmethylpropionat oder Halogenäthylacetat zu dessen entsprechenden Estern umgesetzt wird.

Patentansprüche für das Benennungsland Österreich
- Case 5/812 -

1. Verfahren zur Herstellung neuer Cephalosporine der allgemeinen Formel

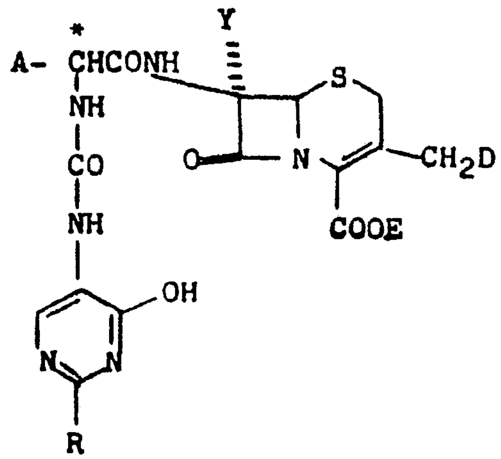

,(I)

bzw.

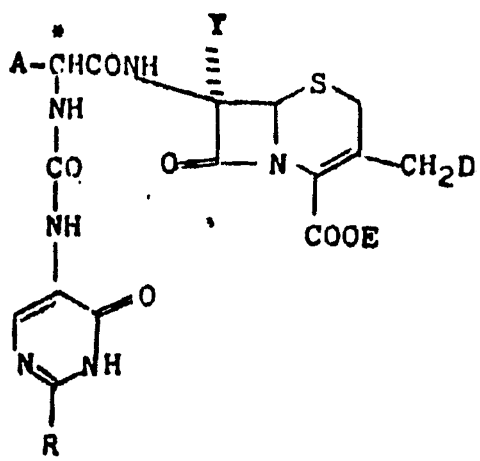

I'

in welchen die Reste A, Y, D, E und R die folgenden Bedeutungen besitzen:

Y ein Wasserstoffatom oder die Methoxygruppe

A sofern Y die Methoxygruppe darstellt, die Phenyl-, 4-Hydroxyphenyl-, 3,4-Dihydroxyphenyl- oder die 2- oder 3-Thienylgruppe, sofern Y ein Wasserstoffatom oder die Methoxygruppe darstellt, eine Gruppe der Formel

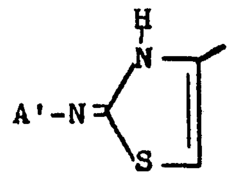

wobei in dieser Gruppe

A' ein Wasserstoffatom, die Gruppe $-COCH_2Cl$, $-COCH_2Br$, $-COOCH_2CCl_3$, die Formyl- oder die Tritylgruppe bedeutet,

D ein Wasserstoffatom, die Acetoxy- oder Aminocarbonyloxygruppe, die Pyridinium- oder 4-Aminocarbonylpyridiniumgruppe, oder die Gruppe -SHet, in welcher Het die 1,3,4-Thiadiazol-5-yl-, 2-Methyl-1,3-4-thiadiazol-5-yl-, die 1,2,4-Thiadiazol-5-yl-, 3-Methyl-1,2,4-thiadiazol-5-yl-, 4H-5,6-Dioxo-1,2,4-triazin-3-yl-, 4-Methyl-5,6-dioxo-1,2,4-triazin-3-yl-, 1-Vinyl-tetrazol-5-yl- oder 1-Allyl-tetrazol-5-ylgruppe oder eine Gruppe der allgemeinen Formel ,

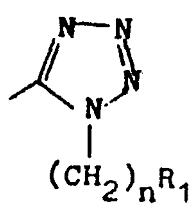

,(II)

bedeutet, in der n die Zahlen 1 bis 3 darstellen und $R_1$ für die Hydroxygruppe, die Amino-, Dimethylamino-, Acetylamino-, Aminocarbonyl-, Aminocarbonylamino-, Aminosulfonyl-, Aminosulfonylamino-, Methylcarbonyl-, Methylsulfonylamino-, Cyano-, Hydroxysulfonylamino-, Methylsulfonyl-, Methylsulfinyl-, sowie für eine Carbonsäure- oder Sulfonsäuregruppe steht, oder in der $(CH_2)_nR_1$ eine Alkylgruppe mit 1 bis 4 Kohlenstoffatomen oder einen 2,3-Dihydroxypropylrest bedeutet,

R eine Gruppe der allgemeinen Formel $-NHR_2$, in der $R_2$ eine substituierte oder unsubstituierte 3-Pyridyl-, 5-Pyrimidinyl-, 2-Thienyl-, 2-Furylmethyl-, 2-Thienylmethyl-, 2-Imidazolylmethyl-, 2-Thiazolylmethyl-, 3-Pyridylmethyl- oder eine 5-Pyrimidinylmethylgruppe darstellt, wobei diese Gruppen durch ein Chloratom, eine Methyl-, Acetylamino-, Hydroxy-, Methylsulfinyl-, Methylsulfonyl-, Aminocarbonyl- oder Aminosulfonylgruppe substituiert sein können,

E ein Wasserstoffatom oder eine in vitro oder in vivo leicht abspaltbare Carboxylschutzgruppe,

desweiteren, sofern D für die Pyridinium- oder Aminocarbonylpyridiniumgruppe steht, von Verbindung an der allgemeinen Formel

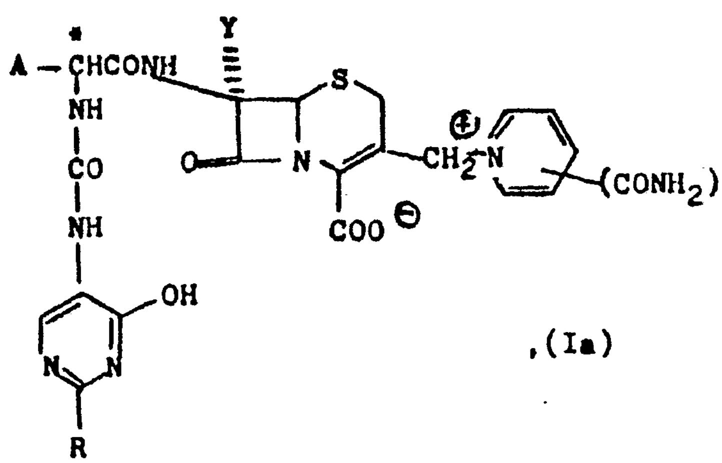

,(Ia)

in der A, R und Y wie oben definiert sind, und, falls E für Wasserstoff steht, von deren Salzen mit anorganischen oder organischen Basen, dadurch gekennzeichnet, daß

a) Zur Herstellung von Verbindungen der allgemeinen Formel I bzw. I', in der D die oben angegebenen Bedeutungen mit Ausnahme der einer Pyridinium- oder Aminocarbonylpyridiniumgruppe besitzt, ein 7-Aminocephalosporansäurederivat der allgemeinen Formel III

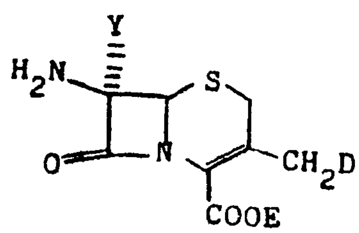

,(III)

in der Y und E wie oben definiert sind und D die oben genannten Bedeutungen mit Ausnahme der einer Pyridinium- oder Aminocarbonylpyridiniumgruppe aufweist, mit einer Ureidocarbonsäure der allgemeinen Formel IV

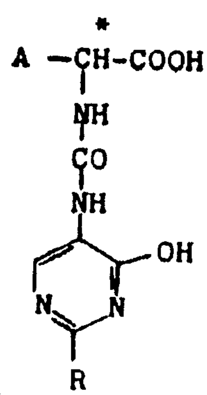

,(IV)

in der A und R die oben genannten Bedeutungen besitzen, oder mit deren Salzen oder reaktiven Derivaten bei Temperaturen zwischen $-40^{o}C$ und $+40^{o}C$ in Gegenwart eines Lösungsmittels und, gegebenenfalls, in Gegenwart einer Base umgesetzt wird, oder

b) eine 7-Aminocephalosporansäure der allgemeinen Formel V

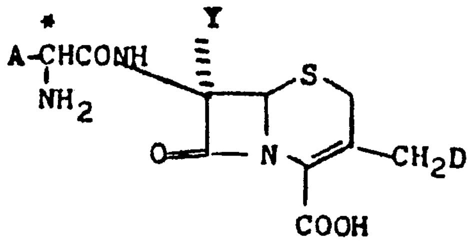

,(V)

in der A, D und Y wie oben definiert sind, oder ihre Salze mit anorganischen oder organischen Basen mit einem Pyrimidinderivat der allgemeinen Formel VI

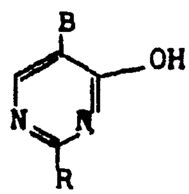

,(VI)

in der R wie oben definiert ist und B die Gruppe -NCO oder ein reaktives Derivat der Gruppe -NHCOOH bzw. die Gruppen -NHCOCl, -NHCOBr oder 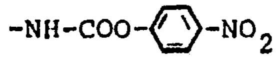 bedeutet oder mit Gemischen solcher Pyrimidine der allgemeinen Formel VI, in der B teils die eine und teils die andere der vorstehend genannten Bedeutungen besitzt, oder in der diese Verbindungen auch teilweise oder ganz als ein den Isocyanaten isomeres 1-(H)-oxazolo(5,4-d)pyrimidin-2-on vorliegen können, in einem Lösungsmittel und einem pH-Bereich zwischen 2,0 und 9,0 bei Temperaturen zwischen -20°C und +50°C umgesetzt wird, oder

c) zur Herstellung von Verbindungen der allgemeinen Formel I bzw. I', in der D entweder die Gruppe -SHet mit den oben für Het angegebenen Bedeutungen oder die Pyridinium- oder Aminocarbonylpyridiniumgruppe bedeutet, und E für Wasserstoff steht, eine Verbindung der allgemeinen Formel VII

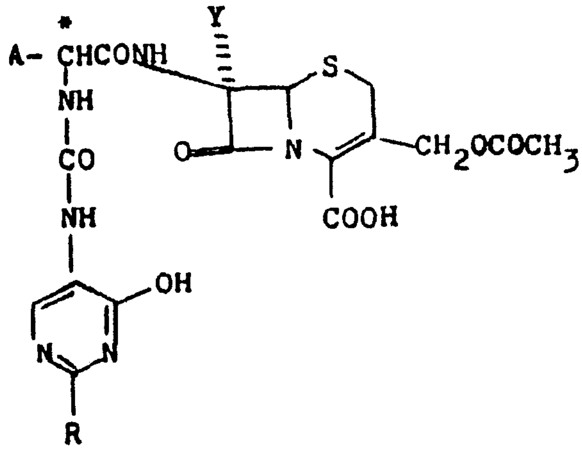
,(VII)

in der A, Y und R wie oben definiert sind, entweder mit einer Verbindung der allgemeinen Formel VIII

Het-S-M ,(VIII)

in der Het die oben angegebenen Bedeutungen hat und M für ein Wasserstoffatom oder ein Alkalimetall oder ein Erdalkalimetall steht oder mit Pyridin bzw. 4-Aminocarbonylpyridin in einem Lösungsmittel bei Temperaturen zwischen $0^{o}$ und $100^{o}C$ in einem pH-Bereich zwischen 2 und 10 zu einer Verbindung der allgemeinen Formel I bzw. I', in der D die Bedeutung einer Gruppe -SHet oder einer Pyridinium- oder 4-Aminocarbonylpyridiniumgruppe und E die eines Wasserstoffatoms besitzt, umgesetzt wird, oder

d) Zur Herstellung einer Verbindung der allgemeinen Formel I bzw. I', in der Y die Methoxygruppe bedeutet, eine Verbindung der allgemeinen Formel IX

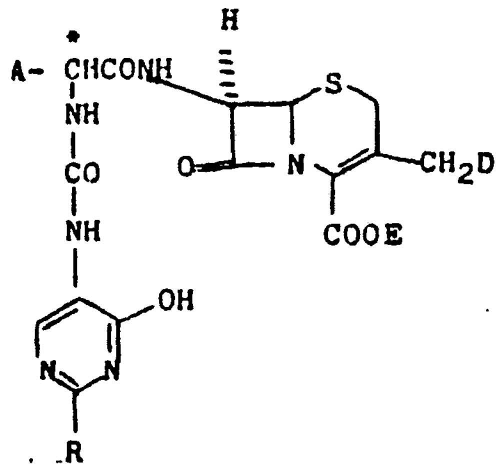
,(IX)

in der A, R, D und E wie oben definiert sind, in Anwesenheit von Methanol mit einem Alkalimetall-Methylat der allgemeinen Formel $M^{+}OCH_3^{-}$, worin $M^{+}$ ein Alkalimetall bedeutet, und dann mit einem Halogenierungsmittel in einem inerten Lösungsmittel bei Temperaturen zwischen $-120^{o}$ und $-10^{o}C$ umgesetzt wird und,
gewünschtenfalls anschließend, eine so erhaltene Verbindung der allgemeinen Formel I bzw. I', worin E eine in vitro oder in vivo leicht abspaltbare Schutzgruppe darstellt, in die freie Carbonsäure der allgemeinen Formel I bzw. I', in der E ein Wasserstoffatom ist, überführt wird und/oder eine Verbindung der allgemeinen Formel I bzw. I', in der E ein Wasserstoffatom ist, in ihre Ester oder, mittels anorganischer oder organischer Basen, in die entsprechenden Salze übergeführt wird.

2. Verfahren zur Herstellung neuer Cephalosporine der allgemeinen Formeln

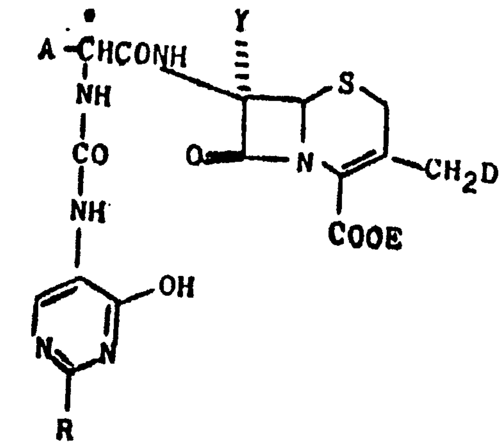

,(I)

bzw.

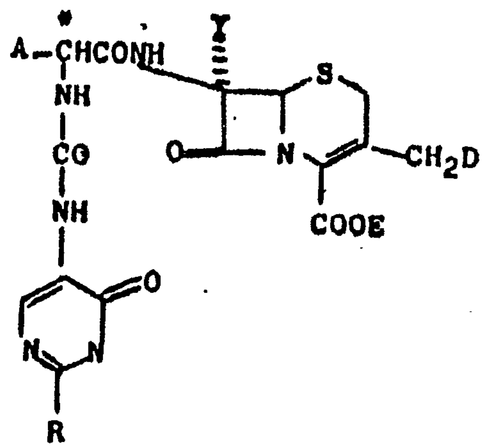

,(I')

in welchen A, Y, D, E und R die folgenden Bedeutungen besitzen:

Y die Methoxygruppe,

A die Phenyl-, 4-Hydroxyphenyl-, 3,4-Dihydroxyphenyl-, oder die 2- oder 3-Thienylgruppe,

D die Acetoxy- oder Aminocarbonyloxygruppe, oder die Gruppe SHet, wobei Het die 1,3,4-Thiadiazol-5-yl-, 2-Methyl-1,3,4-thiadiazol-5-yl-, die 1,2,4-Thiadiazol-5-ylgruppe, die 3-Methyl-1,2,4-thiadiazol-5-ylgruppe, die 4H-5,6-Dioxo-1,2,4-triazin-3-yl- oder 4-Methyl-5,6-dioxo-1,2,4-triazin-3-ylgruppe, die 1-Vinyl-tetrazol-5-yl- oder 1-Allyltetrazol-5-ylgruppe oder eine Gruppe der allgemeinen Formel II

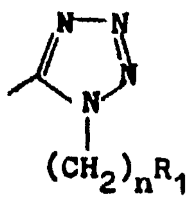 (II)

bedeutet,
in der n die Zahlen 1 bis 3 bedeutet und $R_1$ für die Hydroxygruppe, die Amino-, Dimethylamino-, Acetylamino-, Aminocarbonyl-, Aminocarbonylamino-, Aminosulfonyl-, Aminosulfonylamino-, Methylcarbonyl-, Methylsulfonylamino-, Cyano-, Hydroxysulfonylamino-, Methylsulfonyl-, Methylsulfinyl-, sowie für eine Carbonsäure oder Sulfonsäuregruppe steht, weiter kann $(CH_2)_nR_1$ eine Alkylgruppe mit 1 bis 4 Kohlenstoffatomen oder einen 2,3-Dihydroxypropylrest bedeuten,

R eine Gruppe der Formel $-NHR_2$, worin $R_2$ eine gegebenenfalls substituierte 2-Thienyl-, 2-Furylmethyl-, 2-Thienylmethyl-, 2-Imidazolylmethyl-, 2-Thiazolylmethyl-, 3-Pyridylmethyl-, 5-Pyrimidinylmethyl-, 3-Pyridyl- oder 5-Pyrimidinylgruppe bedeutet, wobei diese Gruppen durch folgende Reste substituiert sein können: ein Chloratom, eine Methyl-, Acetylamino-, Hydroxy-, Methylsulfinyl-, Methylsulfonyl-, Aminocarbonyl- oder Aminosulfonylgruppe,

E ein Wasserstoffatom oder eine in vitro oder in vivo leicht spaltbare Carboxylschutzgruppe und, falls E ein Wasserstoffatom bedeutet, von deren Salzen mit anorganischen oder organischen Basen, dadurch gekennzeichnet, daß

a) eine Ureidocarbonsäure der allgemeinen Formel IV

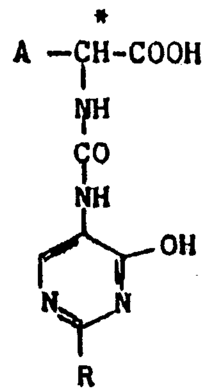

,(IV)

in der A und R die oben angegebenen Bedeutungen besitzen, oder ihre Salze oder reaktiven Derivate, mit 7-Amino-cephalosporansäurederivaten der allgemeinen Formel III

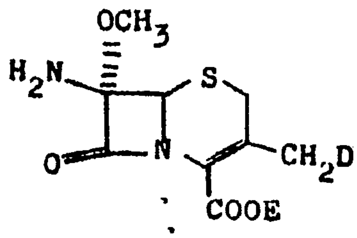

,(III)

in der E wie oben definiert ist, und D die Acetoxy- oder Aminocarbonyloxygruppe oder die Gruppe -SHet bedeutet zwischen $-40^{o}$ und $+40^{o}C$ in Gegenwart eines Lösungsmittels und, gegebenenfalls, einer Base umgesetzt wird oder

b) eine 7-Aminocephalosporansäure der allgemeinen Formel V,

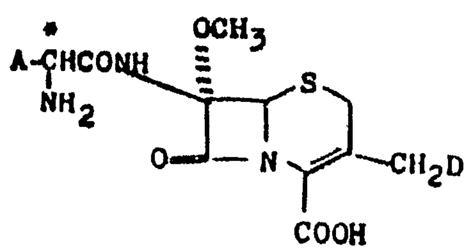

,(V)

in der A,und D wie oben definiert sind, oder ihre Salze mit anorganischen oder organischen Basen mit einem Pyrimidinderivat der allgemeinen Formel VI,

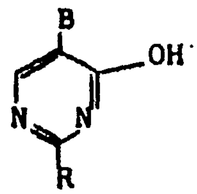

,(VI)

in der R wie oben definiert ist und B die Gruppe -NCO oder ein reaktives Derivat der Gruppe -NHCOOH bzw. die Gruppen -NHCOCl, -NHCOBr oder -NH-COO-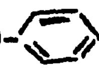-$NO_2$ bedeutet oder mit Gemischen solcher Pyrimidine der allgemeinen Formel III, in der B teils die eine und teils die andere der vorstehend genannten Bedeutungen besitzt, in einem Lösungsmittel und einem pH-Bereich zwischen 2,0 und 9,0 bei Temperaturen zwischen -20°C und +50°C umgesetzt wird, oder

c) zur Herstellung von Verbindungen der allgemeinen Formel I bzw. I', in der D die Gruppe SHet mit den oben für Het angegebenen Bedeutungen bedeutet, und E für Wasserstoff steht, eine Verbindung der allgemeinen Formel

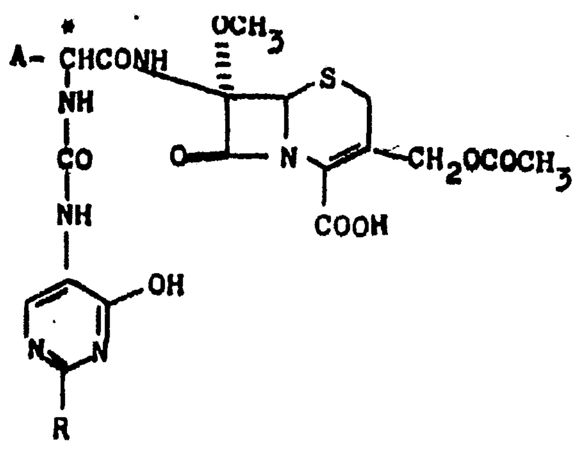

,(VII)

in der A und R die oben angegebenen Bedeutungen haben mit einer Verbindung der allgemeinen Formel

Het-S-M ,(VIII)

in der Het die oben angegebenen Bedeutungen hat und M für ein Wasserstoffatom oder ein Alkalimetall oder ein Erdalkalimetall steht in einem Lösungsmittel bei Temperaturen zwischen 0° und 100°C in einem pH-Bereich zwischen 2 und 10 zu einer Verbindung der allgemeinen Formel I, in der D die Bedeutungen einer -SHetgruppe und E die eines Wasserstoffatoms besitzt, umgesetzt wird oder

d) zur Herstellung einer Verbindung der allgemeinen Formel I bzw. I', in der Y die Methoxygruppe darstellt, eine Verbindung der allgemeinen Formel IX

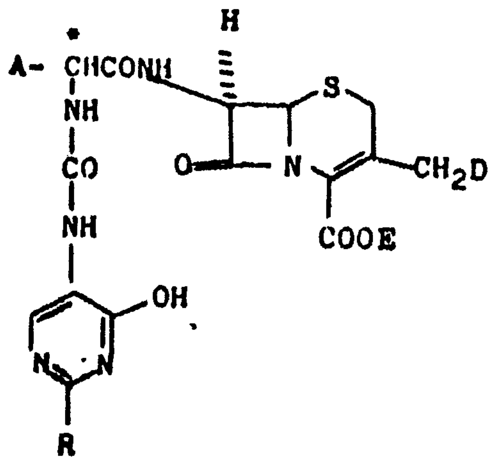

,(IX)

in der A, R, D und E wie oben definiert sind, in Anwesenheit von Methanol mit einem Alkalimetall-Methylat der allgemeinen Formel $M^+OCH_3^-$, worin $M^+$ ein Alkalimetall bedeutet, und dann mit einem Halogenierungsmittel in einem inerten Lösungsmittel bei Temperaturen zwischen -120° und -10°C umgesetzt wird und, gewünschtenfalls anschließend, eine so erhaltene Verbindung der allgemeinen Formeln I oder I', worin E eine in vitro oder in vivo leicht abspaltbare Schutzgruppe darstellt, in die freie Carbonsäure der allgemeinen Formeln I oder I', in der E ein Wasserstoffatom ist, übergeführt wird und/oder eine Verbindung der allgemeinen Formeln I oder I', in der E ein Wasserstoffatom bedeutet, in ihre Ester oder, mittels anorganischen oder organischen Basen, in die entsprechenden Salze übergeführt wird.

3. Verfahren zur Herstellung neuer Cephalosporine der allgemeinen Formeln

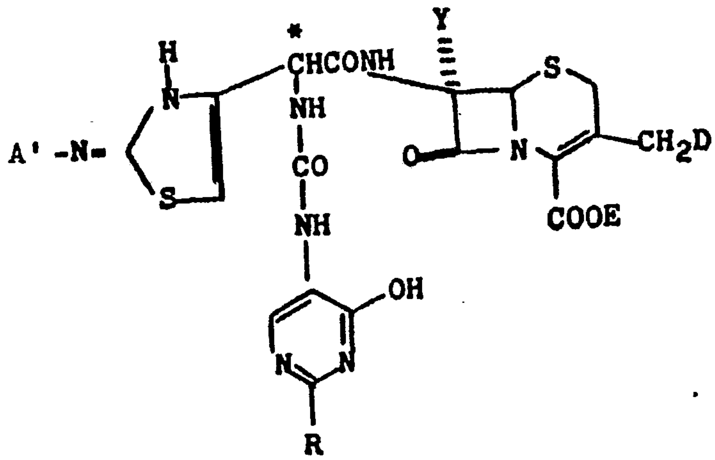

,(I)

bzw.

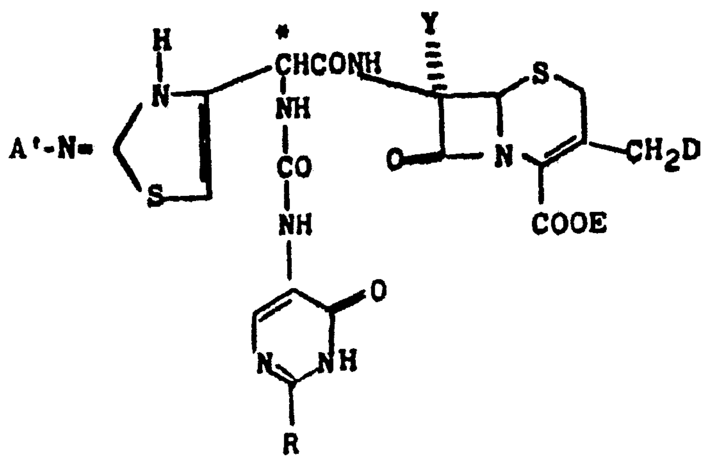

,(I')

in welchen A', Y, E, D und R die folgenden Bedeutungen besitzen:

A' ein Wasserstoffatom, die Gruppe $-COCH_2Cl$, $-COCH_2Br$, $-COOCH_2CCl_3$; die Formyl- oder die Tritylgruppe,

Y, das in der α-Konfiguration steht, ein Wasserstoffatom oder die Methoxygruppe,

D ein Wasserstoffatom, die Acetoxy- oder Aminocarbonyloxygruppe, die Pyridinium- oder Aminocarbonylpyridiniumgruppe oder die Gruppe SHet, wobei Het die 1,3,4-Thiadiazol-5-yl-, 2-Methyl-1,3,4-thiadiazol-5-yl-, die 1,2,4-Thiadiazol-5-ylgruppe oder die 3-Methyl-1,2,4-thiadiazol-5-ylgruppe darstellt,

R eine gruppe der Formel $NHR_2$, worin $R_2$ einen gegebenenfalls substituierten 2-Thienyl-, 2-Furylmethyl-, 2-Thienylmethyl-, 2-Imidazolylmethyl-, 2-Thiazolylmethyl-, 3-Pyridylmethyl-, 5-Pyrimidinylmethyl-, 3-Pyridyl- oder 5-Pyrimidinylrest darstellt, wobei diese Reste wie folgt substituiert sein können:
durch ein Chloratom, eine Methyl-, Acetylamino-, Hydroxy-, Methylsulfinyl-, Methylsulfonyl-, Aminocarbonyl- oder Aminosulfonylgruppe;

E ein Wasserstoffatom oder eine in vitro oder in vivo leicht spaltbare Carboxylschutzgruppe, des weiteren, sofern D für Pyridinium oder Aminocarbonylpyridinium steht, von Verbindungen der allgemeinen Formel Ia

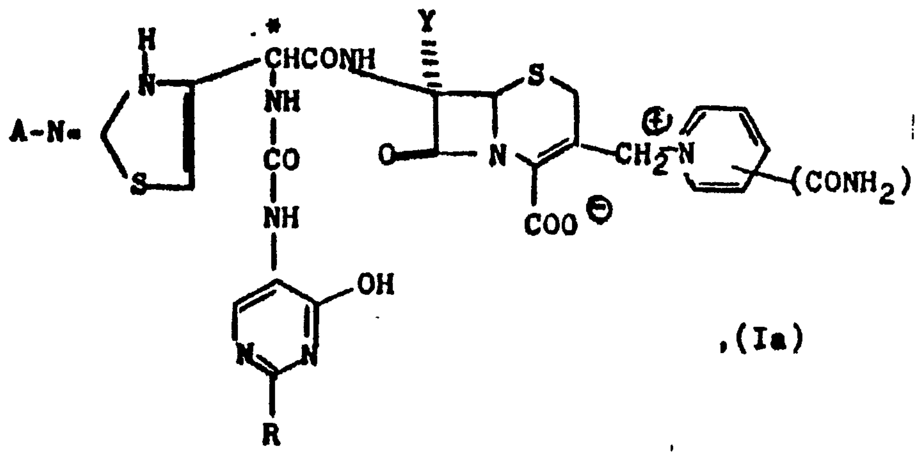

,(Ia)

in der A, Y und R wie oben definiert sind,
und, falls E ein Wasserstoffatom bedeutet, von deren physiologisch verträglichen Salzen mit anorganischen oder organischen Basen, dadurch gekennzeichnet, daß

a) zur Herstellung von Verbindungen der allgemeinen Formel I bzw. I', in der D die oben angegebenen Bedeutungen mit Ausnahme der einer Pyridinium- oder Aminocarbonylpyridiniumgruppe besitzt, eine Ureidocarbonsäure der allgemeinen Formel

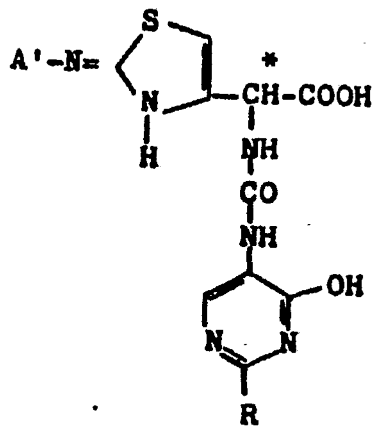

,(IV)

in der A' und R die oben angegebenen Bedeutungen besitzen, oder ihre Salze oder reaktiven Derivate, mit 7-Aminocephalosporansäurederivaten der allgemeinen Formel

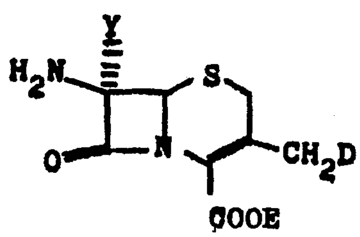

,(III)

in der Y und E wie oben definiert sind und D die oben angegebenen Bedeutungen mit Ausnahme der einer Pyridinium- oder Aminocarbonylpyridiniumgruppe besitzt, zwischen $-40^{\circ}$ und $+40^{\circ}C$ in Gegenwart eines Lösungsmittels und, gegebenenfalls, einer Base umgesetzt wird oder

b) zur Herstellung von Verbindungen der allgemeinen Formeln I,bzw. I', in der D die angegebenen Bedeutungen mit Ausnahme der einer Pyridinium- oder Aminocarbonylpyridiniumverbindung besitzt, eine Verbindung der allgemeinen Formel V,

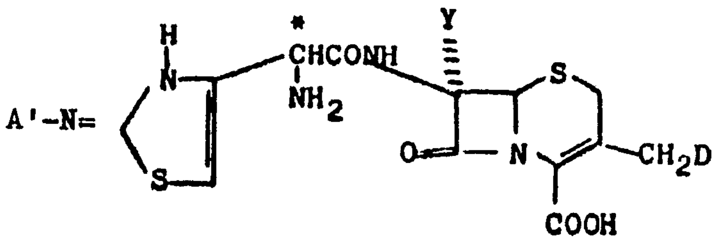
,(V)

in der A' und Y wie oben definiert sind und D die oben angegebenen Bedeutungen mit Ausnahme der einer Pyridinium- oder Aminocarbonylpyridiniumgruppe besitzt, mit einem Pyrimidinderivat der allgemeinen Formel VI,

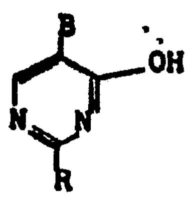
,(VI)

in der R wie oben definiert ist und B die Gruppe -NCO oder ein reaktives Derivat der Gruppe -NHCOOH bzw. die Gruppen -NHCOCl, -NHCOBr oder -NH-COO-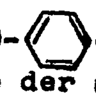-$NO_2$ bedeutet, oder mit Gemischen solcher Pyrimidine der allgemeinen Formel VI, in der B teils die eine und teils die andere der vorstehend genannten Bedeutungen besitzt, in einem Lösungsmittel und einem pH-Bereich zwischen 2,0 und 9,0 bei Temperaturen zwischen -20°C und +50°C umgesetzt wird, oder

c) zur Herstellung von Verbindungen der allgemeinen Formel I bzw. I', in der D entweder die Gruppe SHet mit den oben für Het angegebenen Bedeutungen oder die Pyridinium- oder Aminocarbonylpyridiniumgruppe bedeutet, und E und A für Wasserstoff steht, eine Verbindung der allgemeinen Formel

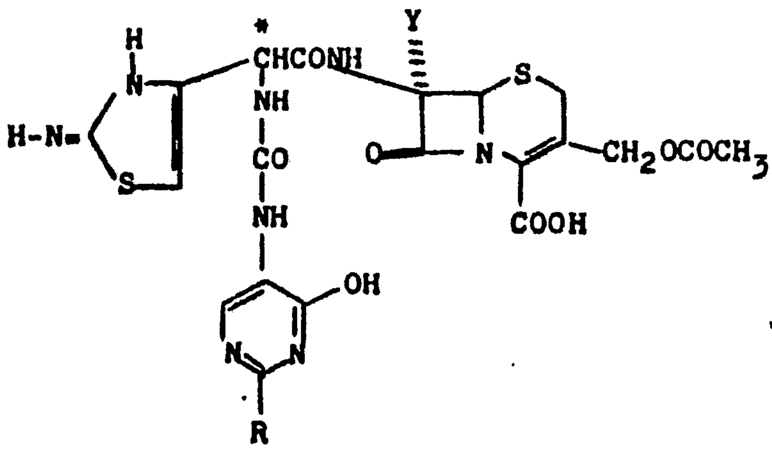

,(VII)

in der R und Y die oben angegebenen Bedeutungen haben, entweder mit einer Verbindung der allgemeinen Formel

Het-S-M ,(VIII)

in der Het die oben angegebenen Bedeutungen hat und M für ein Wasserstoffatom oder ein Alkalimetall oder ein Erdalkalimetall steht oder mit Pyridin bzw. 4-Aminocarbonylpyridin in einem Lösungsmittel bei Temperaturen zwischen 0° und 100°C in einem pH-Bereich zwischen 2 und 10 zu einer Verbindung der allgemeinen Formel I, in der D die Bedeutungen einer -SHet, Pyridinium- oder 4-Aminocarbonylpyridiniumgruppe und E und A die eines Wasserstoffatoms besitzt, umgesetzt wird oder

d) zur Herstellung einer Verbindung der allgemeinen Formel I bzw. I', in der Y die Methoxygruppe darstellt, eine Verbindung der allgemeinen Formel I oder I', in der Y ein Wasserstoffatom bedeutet, in Anwesenheit von Methanol mit einem Alkalimetall-Methylat der allgemeinen Formel $M^+OCH_3^-$, worin $M^+$ ein Alkalimetall bedeutet, und dann mit einem Halogenierungsmittel in einem inerten Lösungsmittel bei Temperaturen zwischen -120 und -10°C umgesetzt wird und gewünschtenfalls anschließend eine so erhaltene Verbindung der allgemeinen Formeln I oder I', worin E eine in vitro oder in vivo leicht abspaltbare Schutzgruppe darstellt, in die freie Carbonsäure der allgemeinen Formeln I oder I', in der E ein Wasserstoffatom ist, übergeführt wird und/oder eine Verbindung der allgemeinen Formeln I oder I', in der E ein Wasserstoffatom bedeutet, in ihre Ester oder, mittels anorganischen oder organischen Basen, in die entsprechenden Salze übergeführt wird.

4. Verfahren gemäß Anspruch 1a, dadurch gekennzeichnet, daß man als reaktive Derivate der Ureidocarbonsäuren der allgemeinen Formel IV deren Säureanhydride, deren reaktive Ester, deren reaktive Amide, deren Säurehalogenide oder Säureazide und als 7-Aminocephalosporansäurederivate der allgemeinen Formel III solche Verbindungen verwendet, bei welchen E die eingangs erwähnten Bedeutungen mit Ausnahme der eines Wasserstoffs besitzt, und die Umsetzung in einem Lösungsmittel, gegebenenfalls in Anwesenheit einer Base und/oder eines Kondensationsmittels erfolgt.

5. Verfahren gemäß Anspruch 1b, dadurch gekennzeichnet, daß eine Verbindung der allgemeinen Formel V oder eines ihrer Salze mit anorganischen oder organischen Basen mit einer Verbindung der allgemeinen Formel VI,

   a) in Wasser oder in mit Wasser mischbaren Lösungsmitteln in Gegenwart von Wasser in einem pH-Bereich von 6,5 bis 8,0 oder
   b) in wasserfreien Lösungsmitteln oder
   c) in einem Gemenge aus Wasser und mit Wasser nicht mischbaren Lösungsmitteln in einem pH-Bereich zwischen 6,5 und 8,0

   umgesetzt wird, oder daß eine Verbindung der allgemeinen Formel V, in der das Wasserstoffatom der Carboxylgruppe durch eine Silylgruppe oder eine andere leicht abspaltbare Schutzgruppe ersetzt ist, mit einer Verbindung der allgemeinen Formel VI in einem wasser- und hydroxylgruppenfreien bzw. aprotischen Lösungsmittel, gegebenenfalls in Gegenwart einer Base, umgesetzt wird.

6. Verfahren gemäß Anspruch 1c, dadurch gekennzeichnet, daß die Umsetzung in einem stark polaren Lösungsmittel bei einem pH-Wert von 4 bis 8 durchgeführt wird.

7. Verfahren gemäß Anspruch 1d, dadurch gekennzeichnet, daß eine Verbindung der allgemeinen Formel IX in einem inerten Mittel gelöst oder suspendiert, mit 2 bis 6 Äquivalenten eines Alkalimetallmethylats in überschüssigem Methanol zusammengebracht und anschließend mit einem Halogenierungsmittel bei Temperaturen zwischen -100 und -50°C umgesetzt unß die Reaktion nach erfolgter Halogenierung durch Säurezusatz abgebrochen wird.

8. Verfahren gemäß Anspruch 1, dadurch gekennzeichnet, daß ein Alkalisalz der Cephalosporansäure der allgemeinen Formeln I oder I' mit einem Pivaloyloxymethylhalogenid oder Halogenmethylacetat, Halogenmethylpropionat oder Halogenäthylacetat zu dessen entsprechenden Estern umgesetzt wird.